# EUROPEAN PATENT SPECIFICATION

(11) **EP 3 801 390 B1**
(45) Date of publication and mention of the grant of the patent: **24.04.2024**
(21) Application number: 19728139.7
(22) Date of filing: 21.05.2019
(51) Int. Cl.: A61F 2/24

(54) **IMPLANTABLE ANNULOPLASTY STRUCTURES TO FIT MULTIPLE ANNULUS SIZES**
IMPLANTIERBARE ANULOPLASTIK-STRUKTUREN ZUR ANPASSUNG MEHRERER RINGRÄUME
STRUCTURES D'ANNULOPLASTIE IMPLANTABLES POUR S'ADAPTER À DE MULTIPLES TAILLES D'ANNEAU

(30) Priority: 24.05.2018 US 201862676145 P; 21.08.2018 US 201862720392 P
(43) Date of publication of application: 14.04.2021
(73) Proprietor: Edwards Lifesciences Innovation (Israel) Ltd., 60376 Or Yehuda (IL)
(72) Inventor: PELEG, Carmel, 6019000 Yehud-Monoson (IL); PEER, Amit, 7646415 Rehovot (IL); KUTZIK, Meir, 5829008 Holon (IL); FOGEL, Alon, 6291407 Tel Aviv (IL); AZOURI, Ben, 6345506 Tel Aviv (IL); BRAUON, Haim, 5020000 Beit Dagan (IL); CHAPPEL-RAM, Shlomit, 4729599 Ramat Hasharon (IL); REICH, Tal, 19130 Moshav Moledet (IL)
(74) Representative: Eisenführ Speiser
(86) International application number: PCT/IL2019/050569
(87) International publication number: WO 2019/224814

(56) References cited:
- US-A1- 2010 030 014
- US-A1- 2013 123 910
- US-A1- 2018 071 098

## Description

### CROSS-REFERENCES TO RELATED APPLICATIONS

The present application claims priority from US Provisional Patent Application 62/676,145 to Peleg et al., filed on May 24, 2018; and US Provisional Patent Application 62/720,392 to Peleg et al., filed on August 21, 2018.

### FIELD OF THE INVENTION

Some applications relate in general to valve repair, for example, repair of an atrioventricular valve of a patient. Some applications relate to minimally invasive and/or transluminal valve repair.

### BACKGROUND

Ischemic heart disease can cause mitral regurgitation by the combination of ischemic dysfunction of the papillary muscles, and the dilatation of the left ventricle that is present in ischemic heart disease, with the subsequent displacement of the papillary muscles and the dilatation of the mitral valve annulus.

Dilation of the annulus of the mitral valve prevents the valve leaflets from fully coapting when the valve is closed. Mitral regurgitation of blood from the left ventricle into the left atrium results in increased total stroke volume and decreased cardiac output, and ultimate weakening of the left ventricle secondary to a volume overload and a pressure overload of the left atrium. Various problems can also occur with regurgitation at the tricuspid valve and other valves.

US 2018/0071098 A1 shows a constricting cord which can be delivered to the vicinity of an annulus using an apparatus that includes a set of support arms, with a respective anchor launcher supported by each of the support arms. An inflatable first balloon is configured to push the support arms away from each other when the first balloon is inflated. An inflatable second balloon is mounted to a shaft and is configured for inflation when the second balloon is disposed distally beyond the first balloon.

US 2013/0123910 A1 shows a delivery device for an annular implant that includes a balloon expansion mechanism and an annular implant having an adjustable dimension. The balloon expansion mechanism includes an inflation tube attached to a non-occluding balloon collar which is supported by trusses radially extending from a trocar. The annular implant further includes a flexible ring core, contiguous coiled spacers, and anchoring blocks. The flexible ring core is adjusted via a cinching mechanism. The anchoring blocks are spaced along the ring core by the contiguous coiled spacers, which keeps the distance between each pair of anchoring blocks equidistant as the ring core diameter is manipulated by the device user.

US 2010/0030014 A1 shows an intracardiac device for restoring the functional elasticity of the cardiac structures, in particular for the treatment of cardiomyopathies and or valvulopathies, by storing energy from the cardiac structures and ceding energy to the cardiac structures during the cardiac cycle, has an elongated shape, is at least partially wound in coils and is attachable to a cardiac structure; the coils are selected in material, number and dimension so as to allow an elastic elongation of the intracardiac device higher than 10% of the rest length of the intracardiac device and are exposed, in use, to the blood flow.

### SUMMARY

The invention is an apparatus comprising and annuloplasty structure as defined in claim 1 and a method performed on a simulation comprising delivering an annuloplasty structure to a simulated annulus to a simulated heart valve as defined in claim 10. This summary is meant to provide some examples and is not intended to be limiting of the scope of the invention in any way. For example, any feature included in an example of this summary is not required by the claims, unless the claims explicitly recite the features. Also, the features, components, steps, concepts, etc. described in examples in this summary and elsewhere in this disclosure can be combined in a variety of ways. The description herein relates to systems, assemblies, methods, devices, apparatuses, combinations, etc. that may be utilized for repairing a valve, such as the mitral valve or tricuspid valve. Various features and steps as described elsewhere in this disclosure may be included in the examples summarized here. The methods, operations, steps, etc. described herein can be performed on a non-living cadaver, cadaver heart, simulator (e.g. with the body parts, tissue, etc. being simulated), etc.

In some applications, a system or an apparatus is provided that comprises an implant structure comprising an annuloplasty structure or annuloplasty ring structure that is configured to fit all or many different annulus sizes. Various systems, apparatuses, and methods are described which enable minimally invasive and/or transluminal (e.g., transcatheter, transvascular, etc.) positioning of the annuloplasty structure along the annulus without having to measure the size of the annulus in advance. In such a manner, systems and apparatuses described herein can accommodate and fits all annulus sizes of any patient or most annulus sizes of most patients.

The implant structure can comprise at least part of an annuloplasty structure (e.g., an annuloplasty ring structure, a partial annuloplasty ring, a full annuloplasty ring, annuloplasty band, etc.) for repairing a dilated valve annulus of a native atrioventricular valve, such as a mitral or tricuspid valve, of a patient.

For some applications, an elongate annuloplasty structure, such as that described above, is transluminally positioned along the annulus and is sequentially attached (e.g., anchored, fastened, clipped, adhered, etc.) thereto. In such applications, the elongate annuloplasty structure can be longer than needed for a given annulus, and thus, the elongate annuloplasty structure comprises apparatus or features which enables shortening of the overall length of the elongate annuloplasty structure while maintaining the body portion of the annuloplasty structure intact (e.g., by not cutting or severing any portion of the body portion). For some applications, the elongate annuloplasty structure is delivered to the annulus in the elongate state, attached (e.g., anchored, etc.) in part to the annulus, and then subsequently to the attaching, shortened. For some applications, the elongate annuloplasty structure is shortened (e.g., compressed) in conjunction with the anchoring process, e.g., alternating between shortening and attaching.

According to the invention which is defined by independent claim 1, the annuloplasty structure is controllably expandable, in part. For such applications, the annuloplasty structure is delivered to the annulus in the shortened state, expanded in part, and subsequently, attached (e.g., anchored, etc.) to the annulus. For some applications, the expanding of the annuloplasty structure occurs in conjunction with the attaching. For example, in such applications, the annuloplasty structure comprises a plurality of attachment sections or anchoring sections alternately disposed with a plurality of controllably-expandable sections. The controllably-expandable sections are controllably expandable by one or more control wires.

There is therefore provided, in accordance with the invention, a system or an apparatus, including an annuloplasty structure. The annuloplasty structure including a body portion (such as an annuloplasty ring body portion, an annuloplasty body portion, etc.) including flexible material. The body portion is shaped and/or configured to define one or more controllably-expandable sections, each one of the one or more controllably-expandable sections having a respective first length. The body portion includes one or more control wires coupled to the one or more controllably-expandable sections, the one or more control wires being movable to facilitate expansion each one of the one or more controllably-expandable sections to assume a respective second length that is greater than the respective first length. The system, apparatus, and/or annuloplasty structure also includes a contracting member and/or a contracting mechanism or attachment mechanism coupled to the contracting member. The contracting member is coupled to the body portion and is configured to contract the body portion independently of the one or more control wires.

In some applications, each one of the one or more controllably-expandable sections includes a part of the flexible material of the body portion, the part being folded, and wherein the one or more control wires is threaded through folds of the part.

In some applications, the part is folded telescopically in a direction away from a longitudinal axis of the body portion at the part.

In some applications, the part is folded in a manner in which at least one pouch is formed between layers of folds of the part, and the pouch has a longitudinal length that is measured along a pouch axis that is generally parallel with a longitudinal axis of the body portion at the part.

In some applications, the one or more control wires is removably coupled to the one or more controllably-expandable sections, and the flexible material is biased such that the one or more controllably-expandable sections expand upon decoupling of the one or more control wires from the one or more controllably-expandable sections.

In some applications, the one or more control wires is fixedly attached to the one or more controllably-expandable sections, and the one or more control wires facilitates expansion of the one or more controllably-expandable sections responsively to pulling on the one or more control wires.

In some applications, the one or more controllably-expandable sections include a plurality of controllably-expandable sections.

In some applications, the one or more control wires includes exactly one control wire which controls all of the plurality of controllably-expandable sections.

In some applications, the one or more control wires includes a plurality of control wires, each one of the plurality of control wires being movable to facilitate expansion of a respective one of the plurality of controllably-expandable sections.

There is additionally provided, in accordance with some applications, a method performed on a simulation, including delivering to a simuilated annulus of a heart valve annuloplasty structure. The annuloplasty structure can be the same as or similar to the annuloplasty structures described above or elsewhere herein. For example, in some applications, the annuloplasty structure includes a body portion (e.g., an annuloplasty ring body portion, etc.) including flexible material, the body portion being shaped and/or configured to define one or more controllably-expandable sections, each one of the one or more controllably-expandable sections having a respective first length. The annuloplasty structure includes one or more control wires coupled to the one or more controllably-expandable sections, the one or more control wires being movable to expand each of the one or more controllably-expandable sections to assume a respective second length that is greater than the respective first length. The annuloplasty structure includes a contracting member and/or a contracting mechanism or attachment mechanism comprising and/or coupled to the contracting member, the contracting member coupled to the body portion and being configured to contract the body portion independently of the one or more control wires. The method further includes controllably expanding the one or more controllably-expandable sections by moving the one or more control wires. This method according to the invention can be performed on a non-living cadaver, cadaver heart, simulator, anthropomorphic ghost, etc.

In some applications the one or more control wires is removably coupled to the one or more controllably-expandable sections, the flexible material is biased such that the one or more controllably-expandable sections expands upon removal of the one or more control wires, and controllably expanding includes decoupling the one or more control wires from the one or more controllably-expandable sections. In some applications, the one or more control wires is fixedly attached to the one or more controllably-expandable sections, and controllably expanding includes pulling on the one or more control wires.

In some applications, the one or more control wires includes exactly one control wire which controls all of the plurality of controllably-expandable sections, and controllably expanding the one or more controllably-expandable sections includes controllably expanding the plurality of controllably-expandable sections by gradually moving the exactly one control wire.

The various systems and/or apparatuses described here and elsewhere herein can further include an adjustment mechanism (e.g., such as on the annuloplasty structure, a catheter, other device, etc.) and contracting the body portion by tensioning the contracting member includes contracting the body portion by tensioning the contracting member by actuating the adjustment mechanism. In some implementations, the adjustment mechanism is coupled to the body portion of the annuloplasty structure and/or to the contracting member. Further, methods herein can include contracting the body portion by tensioning the contracting member, including, for example, contracting the body portion by tensioning the contracting member by actuating the adjustment mechanism.

Other features and steps described elsewhere herein can also be used with the systems, apparatuses, devices, methods, etc. described above. A fuller understanding can be had from the following detailed description of embodiments thereof, taken together with the drawings, in which:

### BRIEF DESCRIPTION OF THE DRAWINGS

Figs. 1-5 and 6A-D are schematic illustrations of respective example annuloplasty structures which comprise controllably-expandable sections;
Fig. 7 is a schematic illustration of an example annuloplasty structure that can be made shorter following anchoring of the annuloplasty structure to tissue of the annulus, in accordance with some applications;
Figs. 8A-B are schematic illustrations of an example annuloplasty structure comprising a deformable section;
Figs. 9-12 are schematic illustrations of respective example annuloplasty structures which comprise respective fabric compositions;
Figs. 13A-C are schematic illustrations of an example system for compressing a portion of an annuloplasty structure;
Figs. 14-15 are respective flow charts of at least some example steps in respective techniques described herein with reference to Figs. 1-13C;
Fig. 16 is a schematic illustration of an example annuloplasty structure which comprises different fabric compositions;
Figs. 17A-F are schematic illustrations of an example annuloplasty structure comprising removable links;
Figs. 18A-C are schematic illustrations of an example system comprising a variably-stretchable annuloplasty ring structure;
Fig. 19 is a schematic illustration of an example system comprising a telescopically-expandable annuloplasty structure; and
Figs. 20, 21A-B, 22A-C, and 23 are schematic illustrations of an example annuloplasty system comprising an example elongate annuloplasty structure.

### DETAILED DESCRIPTION OF EMBODIMENTS

The following description and accompanying figures, which describe and show certain embodiments and examples not part of the invention, are made to demonstrate, in a non-limiting manner, several possible configurations of systems, apparatuses, devices, methods, etc. that may be used for various aspects and features of the present disclosure. As one example, various systems, devices/apparatuses, and methods are described herein, including systems, platforms, devices, methods, etc. that relate to adjustable annuloplasty devices. The scope of the invention is only defined by the claims.

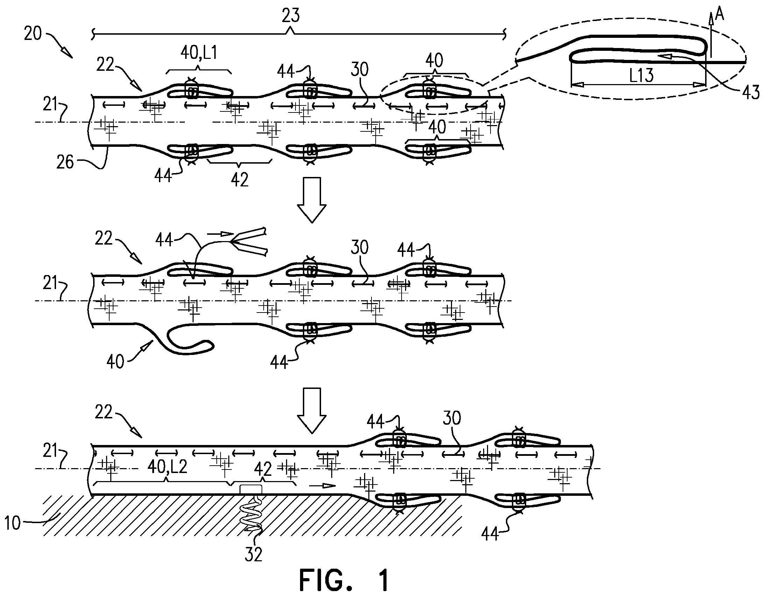

Fig. 1 is a schematic illustration of a system 20 comprising an implant comprising an annuloplasty structure 22 (e.g., an annuloplasty ring structure, an annuloplasty band, a partial annuloplasty ring, a full annuloplasty ring, etc.) which comprises a body portion 23 (e.g., an annuloplasty ring body portion) that is manufactured so that it is shaped and configured so as to define one or more controllably-expandable sections 40, in accordance with some applications. Annuloplasty structure 22 comprises a flexible sleeve 26 shaped and configured so as to define a lumen therethrough. Controllably-expandable sections 40 enable annuloplasty structure 22 to be delivered to an annulus of a cardiac valve of a patient (e.g., typically, but not necessarily a mitral valve or tricuspid valve) in a shortened state. Controllably-expandable sections 40 enable annuloplasty structure 22 to be controllably expanded once inside the body of the patient.

Each controllably-expandable section 40 can comprise a respective part of the flexible material of sleeve 26. During manufacture of sleeve 26, each section 40, which defines the part of sleeve 26 can be folded, or invaginated. Each of these parts can be folded telescopically in a direction A away from a longitudinal axis 21 of annuloplasty structure 22. This direction can be perpendicular to axis 21. Such telescopic folding enables compression of parts of sleeve 26 without affecting an overall inner diameter of sleeve 26, as shown in Fig. 1. Additionally, such telescopic folding enables the subsequent expansion of each section 40 along longitudinal axis 21.

Each of controllably-expandable sections 40, when in a compressed state, can define a longitudinal length L1 of 6-10 mm, e.g., 8 mm, measured longitudinal axis 21 of structure 22. When in an expanded state, each of controllably-expandable sections 40 can define a longitudinal length L2 of 12-18 mm, e.g., 16 mm, measured longitudinal axis 21 of structure 22. Longitudinal length L2 is greater than longitudinal length L1.

Each section 40 can be folded so as to form a pouch 43 between layers of folds of section 40. Pouch 43 can have a longitudinal length L13 of 6.5-9.5 mm, e.g., 7.5 mm, that is measured along a pouch axis that is generally parallel with a longitudinal axis of the annuloplasty ring body portion at the part of the annuloplasty ring body portion that comprises section 40.

Each fold of section 40 can optionally be held together by a knot 44, e.g., a slip knot or any other tie which can come apart easily. For some applications, sleeve 26 is manufactured such that sections 40 are biased such that each section 40 expands passively upon removal of knot 44. For some applications, as shown hereinbelow, each section 40 can be pulled open by a respective control wire.

For some applications, sleeve 26 is expanded once structure 22 is positioned against cardiac tissue.

As illustrated in the bottom figure in Fig. 1, once a given controllably-expandable section 40 is expanded, a tissue anchor 32 or other attachment means can be used to anchor or attach, to cardiac tissue, a section 42 of sleeve 26 adjacent to the now-expanded controllably-expandable section 40. While tissue anchor 32 is depicted in various embodiments and figures herein as a helical anchor that can be rotated or screwed into tissue, other types of tissue anchors or other attachment means can also be used (e.g., flexible anchors, rigid anchors, staples, fasteners, clips, adhesives, sutures, etc.). This sequential anchoring of sleeve 26 to the tissue immediately following the expansion of a first controllably-expandable section 40 but before expansion of a second controllably-expandable section 40 enables the operating physician alternate between expansion and anchoring and enables the operating physician to control the amount of overall expansion of sleeve 26. Once a sufficient amount of sleeve 26 is anchored to annulus tissue 10, the physician can choose to keep the remaining controllably-expandable sections 40 in their compressed states. In such a manner, system 20 minimizes the likelihood of any excess sleeve 26 interfering with the cardiac valve. Additionally, with system 20, the physician does not need to measure the size of the annulus prior to positioning annuloplasty ring structure 22. In such a manner, structure 22 accommodates and fits all native annulus sizes.

For some applications, each section 42 is labeled with a radiopaque marker. For some applications, each section 42 is designated as an anchor-designated section. In the illustrated application, structure 22 comprises a plurality of controllably-expandable sections 40 that are alternately disposed with a plurality of anchor-designated sections 42.

Sleeve 26 can comprise a braided fabric mesh, e.g., comprising polyethylene terephthalate, Dacron (TM), other materials, etc. Body portion 23 (e.g., sleeve 26) can be configured to be placed only partially around a cardiac valve annulus (i.e., to assume a C-shape), and, once anchored in place, to be contracted so as to circumferentially tighten the valve annulus. Optionally, the body portion can be configured to be placed entirely around the valve annulus. In order to tighten the annulus, the system 20 can comprise an actuatable adjustment mechanism or contracting mechanism. The adjustment mechanism or contracting mechanism can be part of and/or coupled to one or more of the annuloplasty structure 22, a flexible elongate contracting member 30 that extends along sleeve 26, and/or another component of the system (e.g., a catheter, etc.). Elongate contracting member 30 can comprise a wire, a ribbon, a rope, or a band, which can comprise a flexible and/or superelastic material, e.g., nitinol, polyester, stainless steel, or cobalt chrome. For some applications, the contracting member 30 comprises a radiopaque material. For some applications, contracting member 30 comprises a braided polyester suture (e.g., Ticron). For some applications, contracting member 30 is coated, such as with polytetrafluoroethylene (PTFE) or another polymer. For some applications, contracting member 30 comprises a plurality of wires that are intertwined to form a rope structure.

The actuatable adjustment mechanism or contraction mechanism can be configured in a variety of ways. For some applications, for example, the actuatable adjustment mechanism comprises a spool or rotating member around which the contracting member can collect. For some applications, the actuatable adjustment mechanism comprises a tensioner that causes a tension force to be applied to the contraction member. For some applications, the actuatable adjustment mechanism comprises a gripper that grips the contraction member in a way that allows a user to apply a tension force to the contraction member. For some applications, the actuatable adjustment mechanism comprises a catheter or multiple catheters configured to interact to draw the contracting member proximally and/or tension it to cause contraction of the annuloplasty structure (e.g., one catheter gripping and allowing pulling of the contraction member, while another catheter provides a resisting force to the annuloplasty structure, etc.). Other applications are also possible.

Once the physician has positioned structure 22 along the annulus and has expanded any number of controllably-expandable sections 40, contracting member 30 is used to contract annuloplasty structure 22. For some applications, contracting member 30 is coupled to an actuatable contracting mechanism as described in US Patent Application Publication 2014/0309661 to Sheps et al., and/or in US Patent Application Publication 2015/0272734 to Sheps et al.. Additionally, implantation of structure 22 can be done using any methods described in the `661 and/or `734 applications.

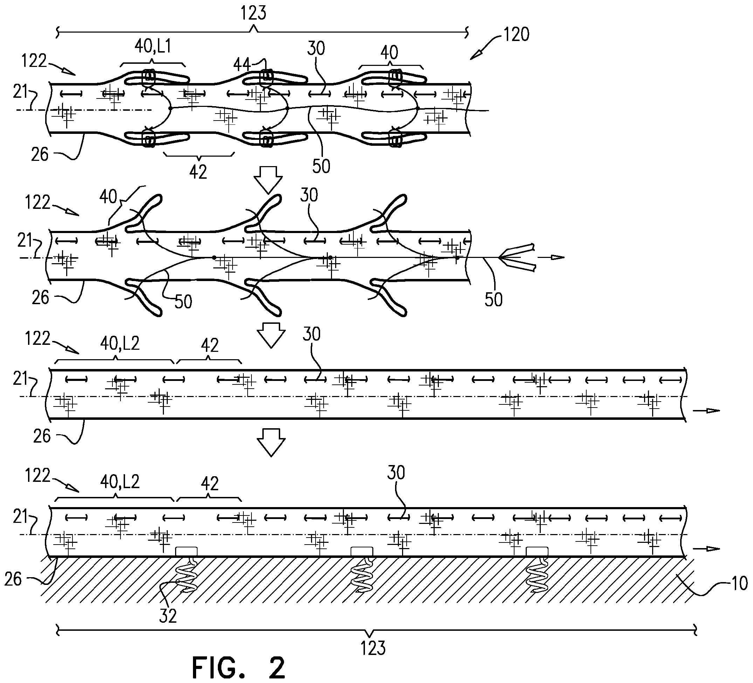

Figs. 2-3 are schematic illustrations of a system 120 comprising an implant comprising a structure 122 (e.g., an annuloplasty structure, an annuloplasty ring structure, etc.) which comprises body portion 123 (e.g., an annuloplasty body portion, an annuloplasty ring body portion, etc.) that is shaped and configured so as to define one or more controllably-expandable sections 40, in accordance with some applications. Except as described below, structure 122 is generally similar to structure 22, described hereinabove with reference to Fig. 1 and like reference numerals refer to like parts.

Structure 122 can comprise a single control wire 50 which is connected to all knots 44 and thereby to all controllably-expandable sections 40 (though multiple control wires can be used in some embodiments). In the illustrated embodiment, once control wire 50 is pulled, as shown, wire 50 releases all of knots 44. Once all of knots 44 are released, sleeve 26 can expand fully and be anchored to tissue. As shown in Fig. 2, once all controllably-expandable sections 40 have been expanded, anchors 32 are used to anchor sleeve 26 to the cardiac tissue. That is, only once sleeve 26 has been expanded, structure 122 is anchored to annulus tissue 10.

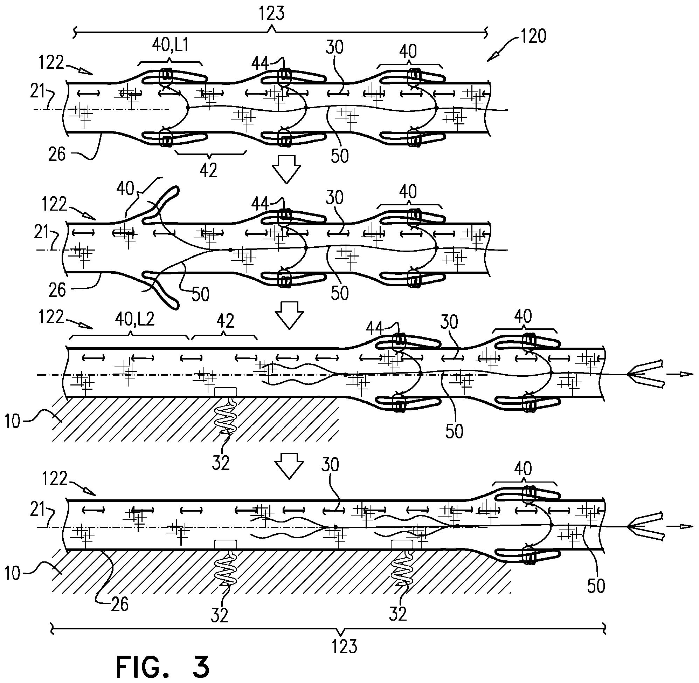

As shown in Fig. 3, control wire 50 can be pulled incrementally and gradually, and a respective anchor 32 or other attachment means can be used to anchor or attach sleeve 26 to the cardiac tissue. This sequential anchoring of sleeve 26 to the tissue immediately following the expansion of a first controllably-expandable section 40 but before expansion of a second controllably-expandable section 40 enables the operating physician alternate between expansion and anchoring and also enables the operating physician to control the amount of overall expansion of sleeve 26. Once a sufficient amount of sleeve 26 is anchored to annulus tissue 10, the physician can choose to keep the remaining controllably-expandable sections 40 in their compressed states. In such a manner, system 120 minimizes the likelihood of any excess sleeve 26 interfering with the cardiac valve. Additionally, with system 120, the physician does not need to measure the size of the annulus prior to positioning annuloplasty ring structure 122. In such a manner, structure 122 accommodates and fits all native annulus sizes.

Once the physician has positioned structure 122 along the annulus and has expanded any number of controllably-expandable sections 40, contracting member 30 is used to contract structure 122. For some applications, contracting member 30 is coupled to and/or is part of an actuatable contracting mechanism or adjustment mechanism, for example, as described elsewhere herein and/or as described in US Patent Application Publication 2014/0309661 to Sheps et al., and/or in US Patent Application Publication 2015/0272734 to Sheps et al.. Additionally, implantation of structure 122 can be done using any methods described in the `661 and/or `734 applications.

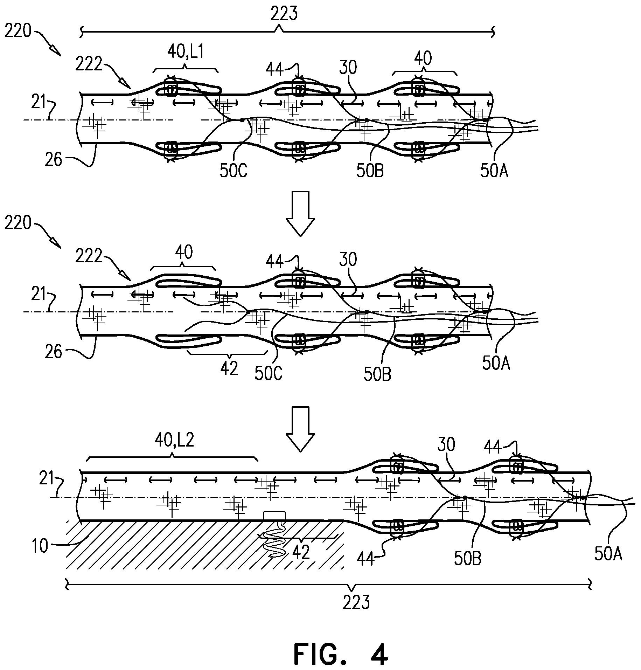

Fig. 4 is a schematic illustration of a system 220 comprising an implant comprising a structure 222 (e.g., an annuloplasty structure, an annuloplasty ring structure, etc.) which comprises a body portion 223 (e.g., an annuloplasty body portion, an annuloplasty ring body portion, etc.) that is shaped and configured so as to define one or more controllably-expandable sections 40, in accordance with some applications. Except as described below, structure 222 is generally similar to structure 22, described hereinabove with reference to Fig. 1 and like reference numerals refer to like parts.

Structure 222 can comprise a plurality of control wires, for example wires 50a, 50b, and 50c. That is, structure 222 can comprise a respective control wire 50 for each controllably-expandable section 40. Each of control wires 50a, 50b, and 50c can be coupled to knots 44 of a respective controllably-expandable section 40. Once each respective control wire 50 is pulled, as shown, wire 50 releases all of knots 44 of the respective controllably-expandable section.

In the illustrated application, once control wire 50a has been pulled, and section 40 expands, a tissue anchor 32 or other attachment means can be used to anchor or attach sleeve 26 to the cardiac tissue at section 42. This sequential anchoring of sleeve 26 to the tissue immediately following the expansion of a first controllably-expandable section 40, but before expansion of a second controllably-expandable section 40 by control wire 50b, enables the operating physician to alternate between expansion and anchoring and enables the operating physician to control the amount of overall expansion of sleeve 26. Once a sufficient amount of sleeve 26 is anchored to annulus tissue 10, the physician can choose to keep the remaining controllably-expandable sections 40 in their compressed states. In such a manner, system 20 minimizes the likelihood of any excess sleeve 26 interfering with the cardiac valve. Additionally, with system 20, the physician does not need to measure the size of the annulus prior to positioning annuloplasty ring structure 222. In such a manner, structure 222 accommodates and fits all native annulus sizes.

Once the physician has positioned structure 222 along the annulus and has expanded any number of controllably-expandable sections 40, contracting member 30 is used to contract annuloplasty structure 222 independently of any remaining control wires 50 coupled to the body portion of structure 222. For some applications, contracting member 30 is coupled to and/or is part of an actuatable contracting mechanism or adjustment mechanism, for example, as described elsewhere herein and/or as described in US Patent Application Publication 2014/0309661 to Sheps et al., and/or in US Patent Application Publication 2015/0272734 to Sheps et al.. Additionally, implantation of structure 222 can be done using any methods described in the `661 and/or `734 applications.

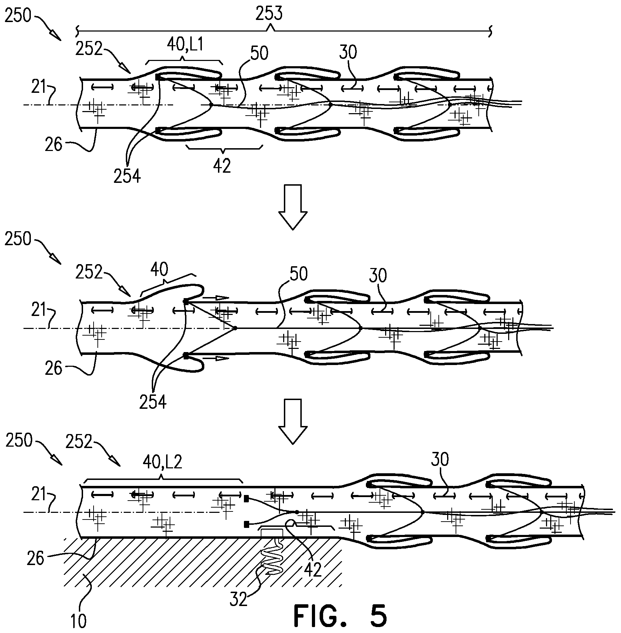

Fig. 5 is a schematic illustration of a system 250 comprising an implant comprising a structure 252 (e.g., an annuloplasty structure, an annuloplasty ring structure, etc.) which comprises a body portion (e.g., an annuloplasty body portion, an annuloplasty ring body portion, etc.) that is shaped and configured so as to define one or more controllably-expandable sections 40, in accordance with some applications. Except as described below, structure 252 is generally similar to structure 22, described hereinabove with reference to Fig. 1 and like reference numerals refer to like parts.

Structure 252 comprises a one or more control wires 50. For some applications, structure 252 comprises a respective control wire 50 for each controllably-expandable section 40. Respective portions of control wire 50 are reversibly coupled directly to material of sleeve 26 at controllably-expandable section 40, at coupling points 254. Once control wire 50 is pulled, as shown, wire 50 actively pulls on the material of sleeve 26 in order to facilitate expansion of controllably-expandable section 40.

As shown in the illustrated application, once control wire 50 has been pulled, and section 40 expands, a tissue anchor 32 or other attachment means can be used to anchor or attach sleeve 26 to the cardiac tissue at section 42. This sequential anchoring of sleeve 26 to the tissue immediately following the expansion of a first controllably-expandable section 40, but before expansion of a second controllably-expandable section 40 by control wire 50, enables the operating physician to alternate between expansion and anchoring and enables the operating physician to control the amount of overall expansion of sleeve 26. Once a sufficient amount of sleeve 26 is anchored to the annulus, the physician can choose to keep the remaining controllably-expandable sections 40 in their compressed states. In such a manner, system 250 minimizes the likelihood of any excess sleeve 26 interfering with the cardiac valve. Additionally, with system 250, the physician does not need to measure the size of the annulus prior to positioning annuloplasty ring structure 252. In such a manner, structure 252 accommodates and fits all native annulus sizes.

Once the physician has positioned structure 252 along the annulus and has expanded any number of controllably-expandable sections 40, contracting member 30 is used to contract annuloplasty structure 252 independently of any remaining control wires 50 coupled to the body portion of structure 252. For some applications, contracting member 30 is coupled to and/or is part of an actuatable contracting mechanism or adjustment mechanism, for example, as described elsewhere herein and/or as described in US Patent Application Publication 2014/0309661 to Sheps et al., and/or in US Patent Application Publication 2015/0272734 to Sheps et al.. Additionally, implantation of structure 252 can be done using any methods described in the `661 and/or `734 applications.

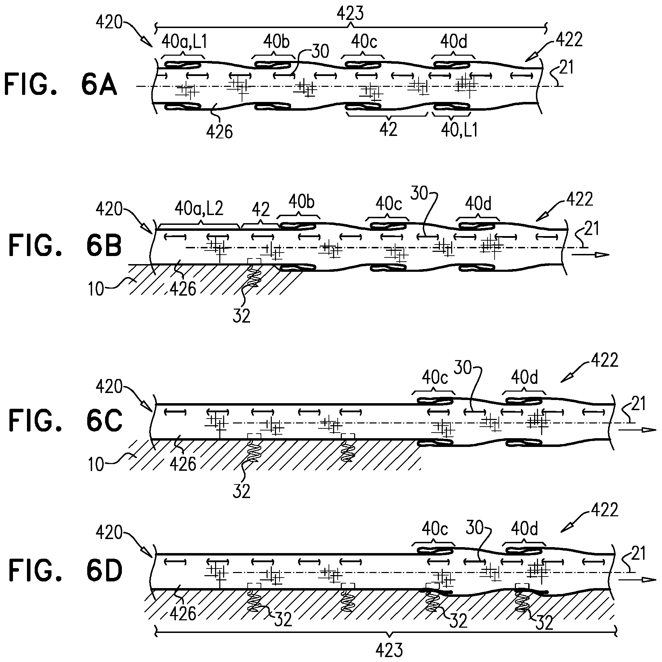

Figs. 6A-D are schematic illustrations of a system 420 comprising an implant comprising a structure 422 (e.g., an annuloplasty structure, an annuloplasty ring structure, etc.) which comprises a body portion (e.g., an annuloplasty body portion, an annuloplasty ring body portion, etc.) that is shaped and configured so as to define one or more controllably-expandable sections 40, in accordance with some applications. Structure 422 comprises a sleeve 426 and a contracting member 30. Except as described below, structure 422 is generally similar to structure 22, described hereinabove with reference to Fig. 1 and like reference numerals refer to like parts.

Sleeve 426 can be manufactured such that sections 40 are biased such that each section 40 retains its folded shape. For example, during manufacture, each section 40 can be ironed, or otherwise set, so as to assume the folded shape. For some applications, as shown hereinbelow, each section 40 can be pulled open by a respective control wire coupled thereto. For some applications, each section 40 can be pulled open by pulling on a different section, e.g., an end, of sleeve 426. Structure 422 is delivered to the annulus in a shortened state, as shown in Fig. 6A, in which controllably-expandable sections 40 assume their respective folded states, each section 40 assuming a length L1.

In Fig. 6B, once structure 422 is positioned along the annulus tissue 10, the physician pulls open a first section 40a and subsequently drives an anchor 32 into tissue 10 at anchor-designated section 42a, so as to retain the now-opened and unfolded state of section 40a, such that section 40a now assumes length L2 that is greater than length L1.

For some applications, each section 42 is labeled with a radiopaque marker. For some applications, each section 42 is designated as an anchor-designated section.

In Fig. 6C, the physician pulls open a second section 40b and subsequently drives an anchor 32 into annulus tissue 10 at anchor-designated section 42b, so as to retain the now-opened and unfolded state of section 40b, such that section 40b now assumes length L2 that is greater than length L1.

In Fig. 6D, the physician determines that the overall length of structure 422 anchored to annulus tissue 10 is sufficient and structure 422 therefore does not need to undergo any further expansion. In order to prevent inadvertent further expansion of sections 40c and 40d, the physician drives a respective tissue anchor 32 through each of sections 40c and 40d so as to retain sections 40c and 40d in their compressed states. Subsequently, contracting member 30 is used to contract structure 422 in order to remodel the shape of the annulus.

This sequential anchoring of sleeve 426 to the tissue immediately following the expansion of a first controllably-expandable section 40a but before expansion of a second controllably-expandable section 40b enables the operating physician to alternate between expansion and anchoring and enables the operating physician to control the amount of overall expansion of sleeve 426. Once a sufficient amount of sleeve 426 is anchored to the annulus, the physician can choose to keep the remaining controllably-expandable sections 40c and 40d in their compressed states. In such a manner, system 420 minimizes the likelihood of any excess sleeve 426 interfering with the cardiac valve. Additionally, with system 420, the physician does not need to measure the size of the annulus prior to positioning annuloplasty ring structure 422. In such a manner, structure 422 accommodates and fits all native annulus sizes.

Sleeve 426 can comprise a braided fabric mesh, e.g., comprising polyethylene terephthalate, Dacron (TM), other materials, etc. Body portion 23 (e.g., sleeve 26) can be configured to be placed only partially around a cardiac valve annulus (i.e., to assume a C-shape), and, once anchored in place, to be contracted so as to circumferentially tighten the valve annulus. Optionally, the body portion can be configured to be placed entirely around the valve annulus. In order to tighten the annulus, the system 420 comprises an actuatable adjustment mechanism or contracting mechanism. The adjustment mechanism or contracting mechanism can be part of and/or coupled to one or more of the annuloplasty structure 422, a flexible elongate contracting member 30 that extends along sleeve 426, and/or another component of the system. Elongate contracting member 30 can comprise a wire, a ribbon, a rope, or a band, which can comprise a flexible and/or superelastic material, e.g., nitinol, polyester, stainless steel, or cobalt chrome. For some applications, the contracting member 30 comprises a radiopaque material. For some applications, contracting member 30 comprises a braided polyester suture (e.g., Ticron). For some applications, contracting member 30 is coated with polytetrafluoroethylene (PTFE). For some applications, contracting member 30 comprises a plurality of wires that are intertwined to form a rope structure. The actuatable adjustment mechanism or contraction mechanism can be configured in a variety of ways, such as in any of the ways described elsewhere herein with respect to other actuatable adjustment mechanisms and/or in other documents.

Once the physician has positioned structure 422 along the annulus and has expanded any number of controllably-expandable sections 40, contracting member 30 is used to contract annuloplasty structure 422. For some applications, contracting member 30 is coupled to and/or is part of an actuatable contracting mechanism or adjustment mechanism, for example, as described elsewhere herein and/or as described in US Patent Application Publication 2014/0309661 to Sheps et al., and/or in US Patent Application Publication 2015/0272734 to Sheps et al.. Additionally, implantation of structure 422 can be done using any methods described in the `661 and/or `734 applications.

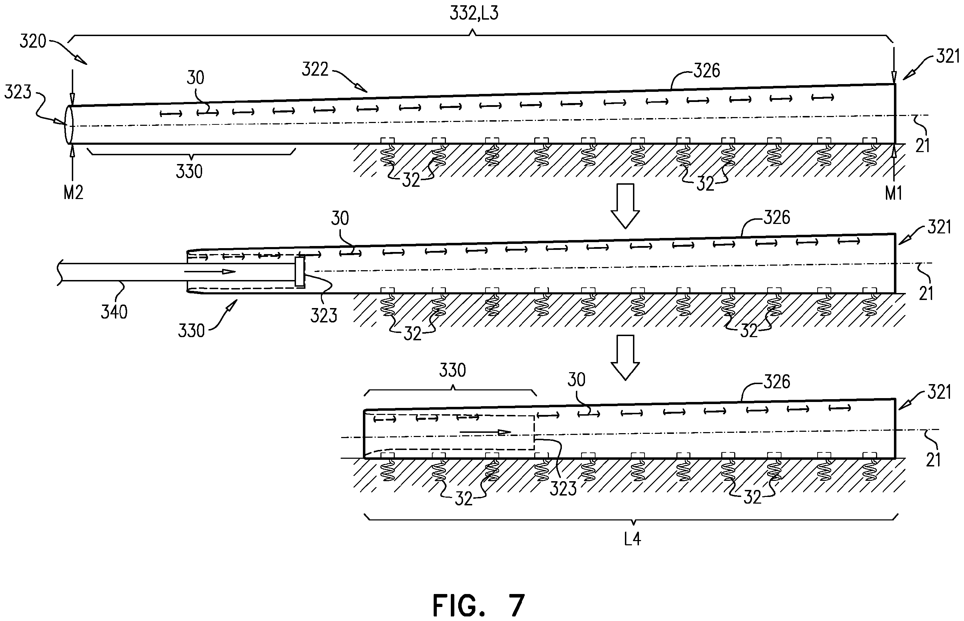

Fig. 7 is a schematic illustration of a system 320 comprising a structure 322 (e.g., an annuloplasty structure, an annuloplasty ring structure, etc.) having a flexible body portion 332 (e.g., an annuloplasty body portion, an annuloplasty ring body portion, etc.) comprising a tapered sleeve 326 and a contracting member 30, in accordance with some applications. Sleeve 326 is shaped and configured so as to define a lumen therethrough. Sleeve 326 is shaped and configured as to define a first end 321 having a first greatest cross-sectional measurement M1 of 4-6 mm, e.g., 5 mm, and a second end 323 having a second greatest cross-sectional measurement M2 of 3.0-3.9 mm, e.g., 3.5 mm, which is smaller than the first greatest cross-sectional measurement.

Any number of tissue anchors 32 or other attachment means can be used to anchor or attach structure 322 to the annulus of the valve. Once a sufficient amount of sleeve 326 is anchored to the annulus, the physician folds, or pushes, the excess portion 330 of sleeve 326 into the lumen of the portion of sleeve 326 that has already been anchored to the annulus, using a pushing tool 340. That is, the physician folds a second end portion of sleeve 326 toward a first end portion of sleeve 326. In such a manner, system 320 minimizes the likelihood of any excess sleeve 326 interfering with the cardiac valve. Additionally, with system 320, the physician does not need to measure the size of the annulus prior to positioning annuloplasty ring structure 322. In such a manner, structure 322 accommodates and fits all native annulus sizes.

For some applications a cutting tool (not shown) can be used to cut excess portion 330 of sleeve 326.

Sleeve 326 can comprise a braided fabric mesh, e.g., comprising polyethylene terephthalate, Dacron (TM), other materials, etc. Body portion 332 (e.g., sleeve 326) can be configured to be placed only partially around a cardiac valve annulus (i.e., to assume a C-shape), and, once anchored in place, to be contracted so as to circumferentially tighten the valve annulus. Optionally, the body portion can be configured to be placed entirely around the valve annulus. In order to tighten the annulus, the system 320 comprises an actuatable adjustment mechanism or contracting mechanism. The adjustment mechanism or contracting mechanism can be part of and/or coupled to one or more of the annuloplasty structure 322, a flexible elongate contracting member 30 that extends along sleeve 326, and/or another component of the system (e.g., a catheter, etc.). Elongate contracting member 30 can comprise a wire, a ribbon, a rope, or a band, which can comprise a flexible and/or superelastic material, e.g., nitinol, polyester, stainless steel, or cobalt chrome. For some applications, the contracting member 30 comprises a radiopaque material. For some applications, contracting member 30 comprises a braided polyester suture (e.g., Ticron). For some applications, contracting member 30 is coated with polytetrafluoroethylene (PTFE). For some applications, contracting member 30 comprises a plurality of wires that are intertwined to form a rope structure. The actuatable adjustment mechanism or contraction mechanism can be configured in a variety of ways, such as in any of the ways described elsewhere herein with respect to other actuatable adjustment mechanisms and/or in other documents.

Once the physician has positioned structure 322 along the annulus and has pushed excess portion 330 within the lumen of the portion of sleeve 326 already anchored to the cardiac tissue, contracting member 30 is used to contract annuloplasty structure 322. For some applications, contracting member 30 is coupled to and/or is part of an actuatable contracting mechanism or adjustment mechanism, for example, as described elsewhere herein and/or as described in US Patent Application Publication 2014/0309661 to Sheps et al., and/or in US Patent Application Publication 2015/0272734 to Sheps et al.. Additionally, implantation of structure 322 can be done using any methods described in the `661 and/or `734 applications.

In such a manner, system 320 facilitates delivering of an elongate structure or annuloplasty ring structure (e.g., structure 322, as shown) to the annulus in an elongate state, anchoring in part to the annulus, and then subsequently to the anchoring, shortening of the elongate structure while maintaining the body portion of the structure or annuloplasty ring structure intact (e.g., by not cutting or severing any portion of the body portion), and subsequently to the shortening, contracting of the structure or annuloplasty ring structure by contracting member 30 in order to contract and remodel the annulus of the patient. In such a manner, the pre-shortening of the structure or annuloplasty ring structure reduces the overall contraction force that is required in order to contract the structure or annuloplasty ring structure.

System 320, therefore, provides a method for (a) delivering to the annulus of the patient structure 322 while body portion 332 has a first longitudinal length L3 of 120-136 mm, (b) subsequently to the delivering, shortening body portion 332 to a second longitudinal length L4 of 40-68 mm independently of contracting member 30, while maintaining the entirety of annuloplasty body portion 332 intact (e.g., no part of portion 332 is cut or severed), and (c) subsequently to the shortening, remodeling the annulus by contracting body portion 332 using contracting member 30. This means that body portion 332 will only need to be contracted along a smaller length, i.e., length L4, rather than along a greater length L3, i.e., if excess portion 330 were not to be pushed within the lumen of sleeve 326. As such, pushing excess portion 330 within the lumen of sleeve 326 reduces the overall force needed to contract body portion 332 once anchored to the annulus.

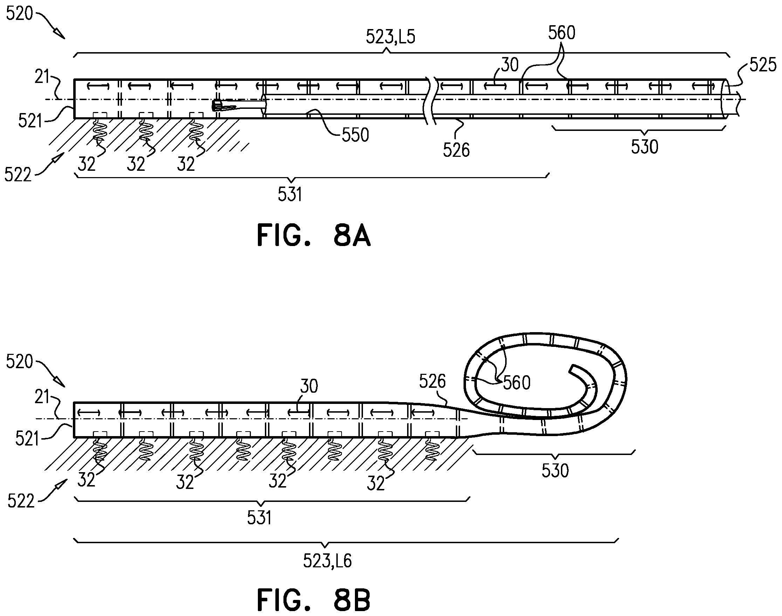

Figs. 8A-B are schematic illustrations of a system 520 comprising a structure 522 (e.g., an annuloplasty structure, an annuloplasty ring structure, etc.) having a flexible body portion 523 (e.g., an annuloplasty body portion, an annuloplasty ring body portion, etc.) comprising (a) a sleeve 526 having an anchor-coupling region or attachment region 531 and a deformable region 530 and (b) a contracting member 30, in accordance with some applications. Sleeve 526 is shaped and configured so as to define a lumen therethrough. Body portion 523 is shaped and configured as to define a first body portion end 521 and a second body portion end 525. Anchor-coupling region or attachment region 531 is disposed between first body portion end 521 and a vicinity of body portion 523 between first and second body portion ends 521 and 525, exclusive of second body portion end 525. Deformable region 530 is disposed between second body portion end 525 and a vicinity of body portion 523 between the first and second body portion ends 521 and 525, exclusive of first body portion end 521. Additionally, body portion 523 can comprise a plurality of radiopaque markers 560 which indicate to the physician where to implant anchors 32.

Optionally, deformable region 530 can comprise a super-elastic material, e.g., nitinol, which enables deformable region 530 to assume its shape. For some applications, the super-elastic material has shape-memory.

Any number of tissue anchors 32 or other attachment means can be used to anchor or attach structure 522 to the annulus of the valve using an anchor-delivery system or attachment system 550. In the illustrated application, anchor-delivery system 550 comprises a tube. While the tube of system 550 is disposed within at least the lumen of deformable region 530, deformable region 530 assumes a first, generally linear shape, as shown in Fig. 8A. Once a sufficient amount of sleeve 526 is anchored to the annulus, the physician removes system 550 from within the lumen of sleeve 526 and deformable region 530 passively assumes a second, deformed shape. As shown, the second, deformed shape is a spiral by way of illustration and not limitation. As such, with system 520, the physician does not need to measure the size of the annulus prior to positioning annuloplasty ring structure 522. In such a manner, structure 522 accommodates and fits all native annulus sizes.

Sleeve 526 can comprise a braided fabric mesh, e.g., comprising polyethylene terephthalate, Dacron (TM), other materials, etc. Body portion 523 (e.g., sleeve 526) can be configured to be placed only partially around a cardiac valve annulus (i.e., to assume a C-shape), and, once anchored in place, to be contracted so as to circumferentially tighten the valve annulus. Optionally, the body portion can be configured to be placed entirely around the valve annulus. In order to tighten the annulus, system 520 comprises an actuatable adjustment mechanism or contracting mechanism. The adjustment mechanism or contracting mechanism can be part of and/or coupled to one or more of the annuloplasty structure 522, a flexible elongate contracting member 30 that extends along sleeve 526, and/or another component of the system (e.g., a catheter, etc.). Elongate contracting member 30 can comprise a wire, a ribbon, a rope, or a band, which can comprise a flexible and/or superelastic material, e.g., nitinol, polyester, stainless steel, or cobalt chrome. For some applications, the contracting member 30 comprises a radiopaque material. For some applications, contracting member 30 comprises a braided polyester suture (e.g., Ticron). For some applications, contracting member 30 is coated with polytetrafluoroethylene (PTFE). For some applications, contracting member 30 comprises a plurality of wires that are intertwined to form a rope structure. The actuatable adjustment mechanism or contraction mechanism can be configured in a variety of ways, such as in any of the ways described elsewhere herein with respect to other actuatable adjustment mechanisms and/or in other documents.

Once the physician has positioned structure 522 along the annulus and allowed deformable region 530 to assume the deformed shape, contracting member 30 is used to contract annuloplasty structure 522. For some applications, contracting member 30 is coupled to and/or is part of an actuatable contracting mechanism or adjustment mechanism, for example, as described elsewhere herein and/or as described in US Patent Application Publication 2014/0309661 to Sheps et al., and/or in US Patent Application Publication 2015/0272734 to Sheps et al.. Additionally, implantation of structure 522 can be done using any methods described in the `661 and/or `734 applications.

In such a manner, system 520 facilitates delivering of an elongate structure or annuloplasty ring structure (e.g., structure 522, as shown) to the annulus in an elongate state, anchoring in part to the annulus, and then subsequently to the anchoring, shortening of the elongate structure while maintaining the body portion of the structure or annuloplasty ring structure intact (e.g., by not cutting or severing any portion of the body portion), and subsequently to the shortening, contracting of the structure or annuloplasty ring structure by contracting member 30 in order to contract and remodel the annulus of the patient. In such a manner, the pre-shortening of the structure or annuloplasty ring structure reduces the overall contraction force that is required in order to contract the structure or annuloplasty ring structure.

System 520, therefore, provides a method for (a) delivering to the annulus of the patient structure 522 while body portion 523 has a first longitudinal length L5 of 122-130 mm, e.g., 125 mm (b) subsequently to the delivering, shortening body portion 523 to a second longitudinal length L6 of 90-129 mm, e.g., 100 mm, independently of contracting member 30, while maintaining the entirety of body portion or annuloplasty body portion 523 intact (e.g., no part of portion 523 is cut or severed), and (c) subsequently to the shortening, remodeling the annulus by contracting body portion 523 using contracting member 30. This means that body portion 523 will only need to be contracted along a smaller length, i.e., length L6, rather than along a greater length L5, i.e., if deformable region were not to be deformed. As such, allowing region 530 to deform reduces the overall force needed to contract body portion 523 once anchored to the annulus.

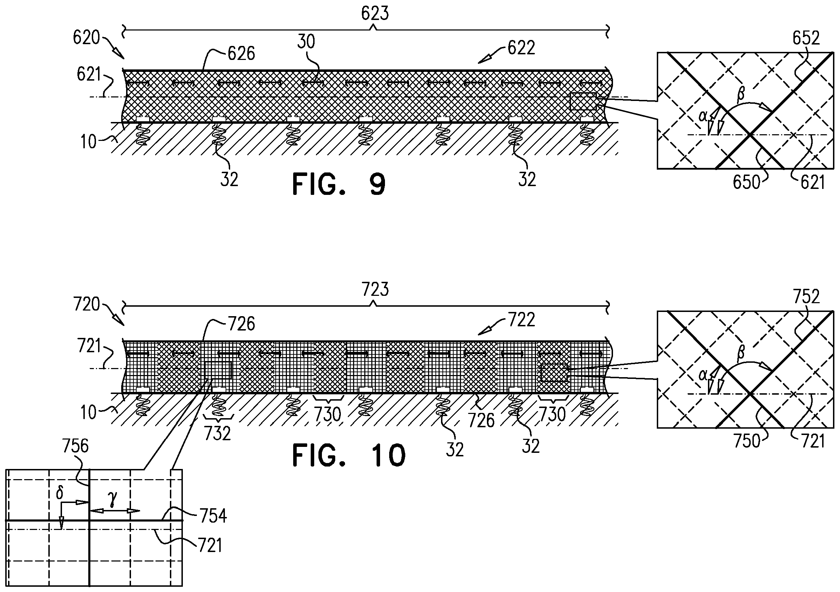

Reference is now made to Fig. 9, which is a schematic illustration of a system 620 comprising a structure 622 (e.g., an annuloplasty structure, an annuloplasty ring structure, etc.) having a flexible body portion 623 (e.g., annuloplasty body portion, an annuloplasty ring body portion, etc.) comprising a sleeve 626 and a contracting member 30, in accordance with some applications. Body portion 623 comprises a sleeve 626. Body portion 623 comprises flexible material comprising a plurality of interwoven fibers.

A first portion 650 of the plurality of interwoven fibers are oriented at an angle α (alpha) of 20 - 70 degrees, e.g., 45 degrees, with respect to a longitudinal axis 621 of body portion 623. A second portion 652 of the plurality of interwoven fibers are oriented at an angle β (beta) of 110 - 160 degrees, e.g., 135 degrees with respect to a longitudinal axis 621 of annuloplasty body portion 623. First and second portions 650 and 652 of interwoven fibers imparts increased longitudinal compressibility and/or stretchability to sleeve 626.

Sleeve 626 can comprise a braided fabric mesh, e.g., comprising polyethylene terephthalate, Dacron (TM), other materials, etc. Body portion 623 (e.g., sleeve 626) can be configured to be placed only partially around a cardiac valve annulus (i.e., to assume a C-shape), and, once anchored in place, to be contracted so as to circumferentially tighten the valve annulus. Optionally, the body portion can be configured to be placed entirely around the valve annulus. In order to tighten the annulus, the system comprises an actuatable adjustment mechanism or contracting mechanism. The adjustment mechanism or contracting mechanism can be part of and/or coupled to one or more of the annuloplasty structure 622, a flexible elongate contracting member 30 that extends along sleeve 626, and/or another component (e.g., catheter, etc.). Elongate contracting member 30 can comprise a wire, a ribbon, a rope, or a band, which can comprise a flexible and/or superelastic material, e.g., nitinol, polyester, stainless steel, or cobalt chrome. For some applications, the contracting member 30 comprises a radiopaque material. For some applications, contracting member 30 comprises a braided polyester suture (e.g., Ticron). For some applications, contracting member 30 is coated with polytetrafluoroethylene (PTFE). For some applications, contracting member 30 comprises a plurality of wires that are intertwined to form a rope structure. The actuatable adjustment mechanism or contraction mechanism can be configured in a variety of ways, such as in any of the ways described elsewhere herein with respect to other actuatable adjustment mechanisms and/or in other documents.

Once the physician has positioned structure 622 along the annulus and has anchored or attached structure 622 to the annulus, contracting member 30 is used to contract annuloplasty structure 622. For some applications, contracting member 30 is coupled to and/or is part of an actuatable contracting mechanism or adjustment mechanism, for example, as described elsewhere herein and/or as described in US Patent Application Publication 2014/0309661 to Sheps et al., and/or in US Patent Application Publication 2015/0272734 to Sheps et al.. Additionally, implantation of structure 622 can be done using any methods described in the `661 and/or `734 applications.

Reference is now made to Fig. 10, which is a schematic illustration of a system 720 comprising a structure 722 (e.g., an annuloplasty structure, an annuloplasty ring structure, etc.) having a flexible body portion 723 (e.g., annuloplasty body portion, an annuloplasty ring body portion, etc.) comprising a sleeve 726 and a contracting member 30, in accordance with some applications. Body portion 723 comprises a sleeve 726. Body portion 723 comprises flexible material comprising first and second longitudinal sections 730 and 732 which alternate along a longitudinal length of body portion 723.

First longitudinal section 730 comprises flexible material comprising a plurality of interwoven fibers. A first portion 750 of the plurality of interwoven fibers are oriented at an angle α (alpha) of 20 - 70 degrees, e.g., 45 degrees, with respect to a longitudinal axis 721 of annuloplasty ring body portion 723. A second portion 752 of the plurality of interwoven fibers are oriented at an angle β (beta) of 110 - 160 degrees, e.g., 135 degrees with respect to a longitudinal axis 721 of annuloplasty ring body portion 723. First and second portions 750 and 752 of the interwoven fibers imparts increased longitudinal compressibility and/or stretchability to first longitudinal section 730.

Second longitudinal section 732 comprises flexible material comprising a plurality of interwoven fibers. A third portion 754 of the plurality of interwoven fibers are oriented at an angle γ (gamma) of 0 - 15 degrees, e.g., 0 degrees or 180 degrees, with respect to a longitudinal axis 721 of annuloplasty body portion 723. A fourth portion 756 of the plurality of interwoven fibers are oriented at an angle δ (delta) of 75 - 90 degrees, e.g., 90 degrees with respect to a longitudinal axis 721 of annuloplasty body portion 723. Third and fourth portions 754 and 756 of the interwoven fibers imparts increased strength, rigidity, and/or stability to second longitudinal section 732. Thus, each of second longitudinal sections 732 is designated for driving a respective tissue anchor 32 therethrough, i.e., sections 732 are anchor-designated sections.

Therefore, there is typically a trade-off between (i) strength, rigidity, and/or stability, and (ii) compressibility and/or stretchability. By providing both (i) anchor-designated sections 732 with increased strength, rigidity, and/or stability specifically where anchors will be anchored, and (ii) sections 730 with increased compressibility and/or stretchability specifically where anchors will not be anchored, structure 722 is provided with both (i) sufficient strength, rigidity, and/or stability for anchors 32 to be fastened thereto, and (ii) sufficient compressibility and/or stretchability for adapting to various annulus sizes.

Sleeve 726 can comprise a braided fabric mesh, e.g., comprising polyethylene terephthalate, Dacron (TM), other materials, etc. Body portion 723 (e.g., sleeve 726) can be configured to be placed only partially around a cardiac valve annulus (i.e., to assume a C-shape), and, once anchored in place, to be contracted so as to circumferentially tighten the valve annulus. Optionally, the body portion can be configured to be placed entirely around the valve annulus. In order to tighten the annulus, the system comprises an actuatable adjustment mechanism or contracting mechanism. The adjustment mechanism or contracting mechanism can be part of and/or coupled to one or more of the annuloplasty structure 722, a flexible elongate contracting member 30 that extends along sleeve 726, and/or another component (e.g., catheter, etc.). Elongate contracting member 30 can comprise a wire, a ribbon, a rope, or a band, which can comprise a flexible and/or superelastic material, e.g., nitinol, polyester, stainless steel, or cobalt chrome. For some applications, the contracting member 30 comprises a radiopaque material. For some applications, contracting member 30 comprises a braided polyester suture (e.g., Ticron). For some applications, contracting member 30 is coated with polytetrafluoroethylene (PTFE). For some applications, contracting member 30 comprises a plurality of wires that are intertwined to form a rope structure. The actuatable adjustment mechanism or contraction mechanism can be configured in a variety of ways, such as in any of the ways described elsewhere herein with respect to other actuatable adjustment mechanisms and/or in other documents.

Once the physician has positioned structure 722 along the annulus and has anchored or attached structure 722 to the annulus, contracting member 30 is used to contract annuloplasty structure 722. For some applications, contracting member 30 is coupled to and/or is part of an actuatable contracting mechanism or adjustment mechanism, for example, as described elsewhere herein and/or as described in US Patent Application Publication 2014/0309661 to Sheps et al., and/or in US Patent Application Publication 2015/0272734 to Sheps et al.. Additionally, implantation of structure 722 can be done using any methods described in the `661 and/or `734 applications.

Thus, first longitudinal sections 730 have a greater degree of compressibility than second longitudinal sections 732. As such, first longitudinal sections 730 reduce the overall compressible force to body portion 723 by contracting member 30 during contraction of structure 722.

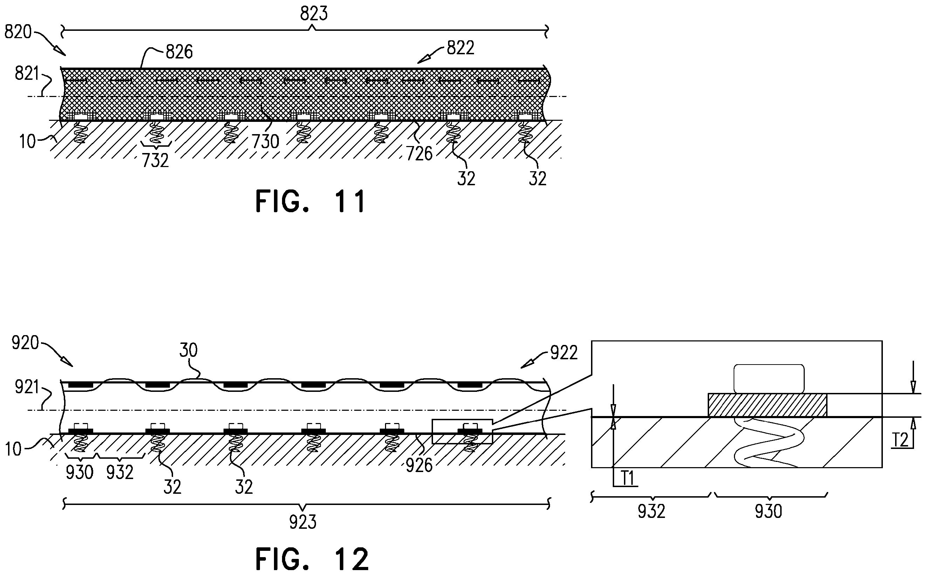

Reference is now made to Fig. 11, which is a schematic illustration of a system 820 comprising a structure 822 (e.g., an annuloplasty structure, an annuloplasty ring structure, etc.) having a flexible body portion (e.g., an annuloplasty body portion, an annuloplasty ring body portion, etc.) comprising a sleeve 826 and a contracting member 30, in accordance with some applications. Body portion 823 comprises flexible material comprising first and second longitudinal sections 730 and 732 which alternate along a longitudinal length of body portion 823 and along a longitudinal axis 821 of structure 822. Except as described below, structure 822 is generally similar to structure 722, described hereinabove with reference to Fig. 10 and like reference numerals refer to like parts.

As shown in Fig. 11, sleeve 826 defines second longitudinal sections 732 only at an area through which tissue anchor 32 is deployed, i.e., at a portion of the lateral wall of sleeve 826 designated to rest against annulus tissue 10. Sections 732 can be disposed opposite contracting member 30. Unlike in Fig. 10 which shows each one of sections 732 occupying 360 degrees of the circumference of sleeve 726, each section 732 of structure 822 of Fig. 11 occupies 75 - 180 degrees of the circumference of sleeve 826. In such a manner, a majority of sleeve 826 comprises sections 730, and thus, sleeve 826 exhibits a high degree of compressibility and/or expandability.

Once the physician has positioned structure 822 along the annulus and has anchored or attached structure 822 to the annulus, contracting member 30 is used to contract annuloplasty structure 822. For some applications, contracting member 30 is coupled to and/or is part of an actuatable contracting mechanism or adjustment mechanism, for example, as described elsewhere herein and/or as described in US Patent Application Publication 2014/0309661 to Sheps et al., and/or in US Patent Application Publication 2015/0272734 to Sheps et al.. Additionally, implantation of structure 822 can be done using any methods described in the `661 and/or `734 applications.

Reference is now made to Fig. 12, which is a schematic illustration of a system 920 comprising a structure 922 (e.g., an annuloplasty structure, an annuloplasty ring structure, etc.) having a flexible body portion 923 (e.g., an annuloplasty body portion, an annuloplasty ring body portion, etc.) comprising sleeve 926 and a contracting member 30, in accordance with some applications. Body portion 923 comprises flexible material comprising first and second longitudinal sections 932 and 930 which alternate along a longitudinal length of body portion 923 and along a longitudinal axis 921 of structure 922.

Each one of first sections 932 defines a compressible section having compressible-section wall having a first thickness T1 of 0.05-0.1 mm, e.g., 0.07 mm. Each one of second sections 930 defines an anchor-designated section having an anchor-designated wall having a second thickness T2 of 0.15-0.2 mm, e.g., 0.18 mm. Second thickness T2 is greater than first thickness T1 and second thickness T2 is such so as to impart rigidity and/or stability to the respective sections 930 of sleeve 926 though which a respective anchor 32 is deployed. For some applications, each section 930 is labeled with a radiopaque marker.

First longitudinal sections 932, due to their respective thickness, have a greater degree of compressibility than second longitudinal sections 930. As such, first longitudinal sections 932 reduce the overall compressible force to body portion 923 by contracting member 30 during contraction of structure 922.

Sleeve 926 can comprise a braided fabric mesh, e.g., comprising polyethylene terephthalate, Dacron (TM), other materials, etc. Body portion 923 (e.g., sleeve 926) can be configured to be placed only partially around a cardiac valve annulus (i.e., to assume a C-shape), and, once anchored in place, to be contracted so as to circumferentially tighten the valve annulus. Optionally, the body portion can be configured to be placed entirely around the valve annulus. In order to tighten the annulus, the system comprises an actuatable adjustment mechanism or contracting mechanism. The adjustment mechanism or contracting mechanism can be part of and/or coupled to one or more of the annuloplasty structure 922, a flexible elongate contracting member 30 that extends along sleeve 926, and/or another component (e.g., catheter, etc.). Elongate contracting member 30 can comprise a wire, a ribbon, a rope, or a band, which can comprise a flexible and/or superelastic material, e.g., nitinol, polyester, stainless steel, or cobalt chrome. For some applications, the contracting member 30 comprises a radiopaque material. For some applications, contracting member 30 comprises a braided polyester suture (e.g., Ticron). For some applications, contracting member 30 is coated with polytetrafluoroethylene (PTFE). For some applications, contracting member 30 comprises a plurality of wires that are intertwined to form a rope structure. The actuatable adjustment mechanism or contraction mechanism can be configured in a variety of ways, such as in any of the ways described elsewhere herein with respect to other actuatable adjustment mechanisms and/or in other documents.

Once the physician has positioned structure 922 along the annulus and has anchored or attached structure 922 to the annulus, contracting member 30 is used to contract annuloplasty structure 922. For some applications, contracting member 30 is coupled to and/or is part of an actuatable contracting mechanism or adjustment mechanism, for example, as described elsewhere herein and/or as described in US Patent Application Publication 2014/0309661 to Sheps et al., and/or in US Patent Application Publication 2015/0272734 to Sheps et al.. Additionally, implantation of structure 922 can be done using any methods described in the `661 and/or `734 applications.

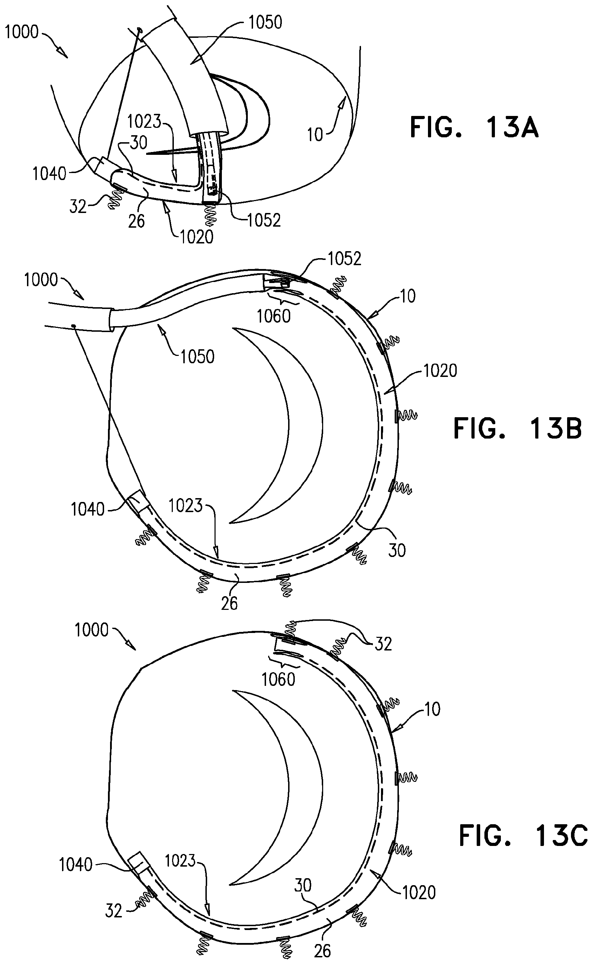

Reference is now made to Figs. 13A-C, which are schematic illustrations of a system 1000 comprising a structure 1020 (e.g., an annuloplasty structure, an annuloplasty ring structure, etc.) having a flexible body portion 1023 (e.g., an annuloplasty body portion, an annuloplasty ring body portion, etc.) comprising a sleeve 26 and a contracting member 30, in accordance with some applications. It is to be noted that annuloplasty structure 1020 can comprise any annuloplasty structure or annuloplasty ring structure described herein with reference to Figs. 1-12. Additionally, structure 1020 can comprise a contracting mechanism 1040 comprising and/or coupled to contracting member 30 as described in US Patent Application Publication 2014/0309661 to Sheps et al., and/or in US Patent Application Publication 2015/0272734 to Sheps et al.. Additionally, implantation of structure 1020 can be done using any methods described in the `661 and/or `734 applications.

In Fig. 13A, an anchor-delivery system 1050 or other attachment system can be used to anchor or attach a first section of annuloplasty body portion 1023 of annuloplasty structure 1020, for example, by deploying one or more tissue anchors 32 through sleeve 26 and into annulus tissue 10, from within a lumen of sleeve 26.

In Fig. 13B, anchor-delivery system 1050 is used to compress a second section 1060 of annuloplasty body portion 1023 toward the first section of annuloplasty body portion 1023. For some applications, system 1050 can reversibly engage and be reversibly coupled to a portion of sleeve 26 during the compressing of second section 1060. For example, an anchor driver 1052 can reversibly ensnare a portion of sleeve 26.

In Fig. 13C, anchor-delivery system 1050 is used to drive a tissue anchor 32 through the now-compressed second section 1060 of body portion 1023. For some applications, the tissue anchor can be delivered downstream of section 1060. It is to be noted that Figs. 13A-C show compression of only a single section 1060 of body portion 1023 by way of illustration and not limitation. Sequential anchoring or attachment of sleeve 26 to the tissue can be done immediately or shortly following the compression respective sections of body portion 1023 which enables the operating physician alternate between compression and anchoring and also enables the operating physician to control the amount of overall compression of sleeve 26. In such a manner, system 1000 minimizes the likelihood of any excess sleeve 26 interfering with the cardiac valve. Additionally, with system 1000, the physician does not need to measure the size of the annulus prior to positioning annuloplasty structure 1020. In such a manner, structure 1020 accommodates and fits all native annulus sizes.

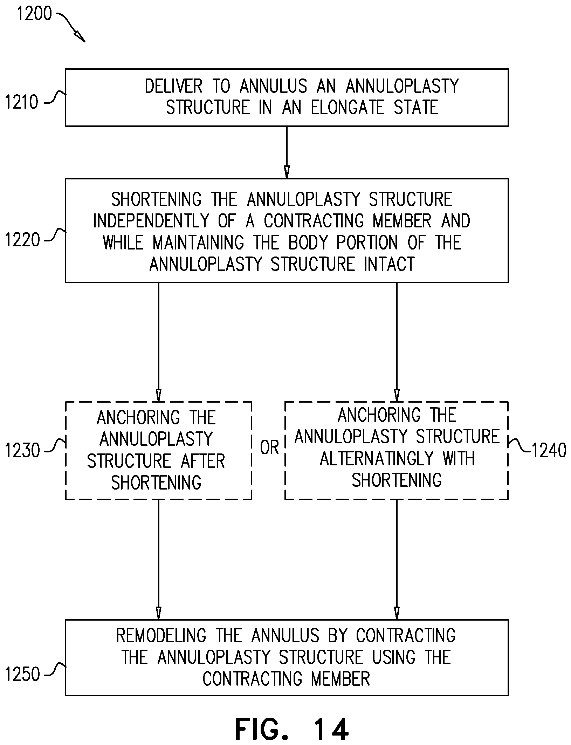

Figs. 14-15 are respective flow charts of at least some steps in respective techniques described herein with reference to Figs. 1-13C, in accordance with respective applications.

Fig. 14 shows a method 1200 for delivering a structure (e.g., an annuloplasty structure, an annuloplasty ring structure, etc.) in an elongate state and subsequently shortening the structure prior to contraction. In step 1210, a structure or "annuloplasty structure" (which can be any of the annuloplasty structures or similar structures described herein) is delivered to the annulus and/or heart atrium in an elongate state. In step 1220, the annuloplasty structure is shortened independently of contracting member 30, while maintaining the body portion of the annuloplasty structure intact (e.g., by not cutting or severing any portion of the body portion).

For some applications, the annuloplasty structure is anchored to the annulus tissue only once the structure has been shortened in accordance with the decision of the operating physician (step 1230). Optionally, the annuloplasty structure is shortened alternatingly with anchoring, as per step 1240. That means, the annuloplasty structure can be shortened in part, then anchored to the annulus tissue using a first tissue anchor. Subsequently, the annuloplasty structure can be shortened in part, again, then anchored to the annulus tissue using a second tissue anchor. Finally, in step 1250, the annulus is remodeled by contracting the annuloplasty structure using the contracting member 30.

Reference is now made to Figs. 7, 8, 13A-C, 14, and 17A-F. It is to be noted that method 1200 described herein with reference to Fig. 14 can be applied to systems and apparatuses described herein with reference to Figs. 7, 8, 13A-C, and 17A-F.

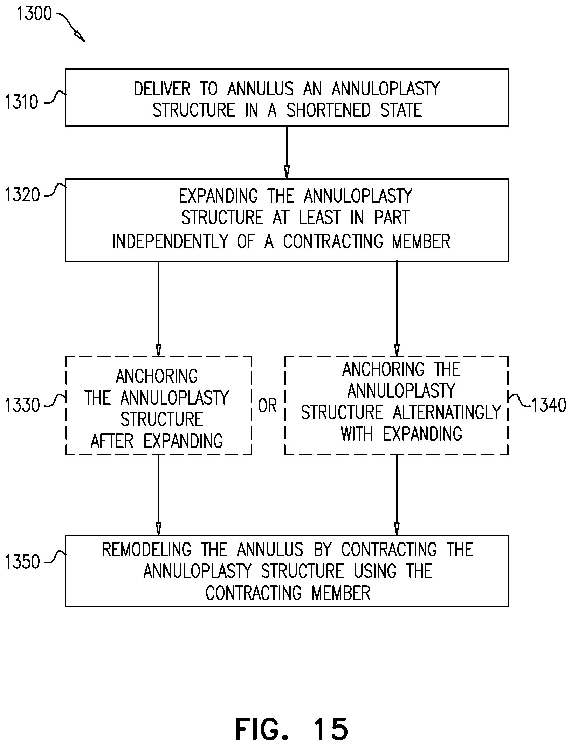

Fig. 15 shows a method 1300 for delivering a structure (e.g., an annuloplasty structure, an annuloplasty ring structure, etc.) in a shortened state and subsequently expanding the structure prior to contraction. In step 1310, an annuloplasty structure (which can be any of the annuloplasty structures or similar structures described herein) is delivered to the annulus and/or heart atrium in a shortened state. In step 1320, the annuloplasty structure is expanded at least in part independently of contracting member 30, while maintaining the body portion of the annuloplasty structure intact (e.g., by not cutting or severing any portion of the body portion).

For some applications, the annuloplasty structure is anchored to the annulus tissue only once the structure has been expanded in accordance with the decision of the operating physician (step 1330). Optionally, the annuloplasty structure is expanded alternatingly with anchoring, as per step 1340. That means, the annuloplasty structure can be expanded in part, then anchored to the annulus tissue using a first tissue anchor. Subsequently, the annuloplasty structure can be expanded in part, again, then anchored to the annulus tissue using a second tissue anchor. Finally, in step 1350, the annulus is remodeled by contracting the annuloplasty structure using the contracting member 30.

Reference is now made to Figs. 1-5, 6A-D, 15, 18A-C, and 19. It is to be noted that method 1200 described herein with reference to Fig. 15 can be applied to systems and apparatuses described herein with reference to Figs. 1-5, 6A-D, 18A-C, and 19.

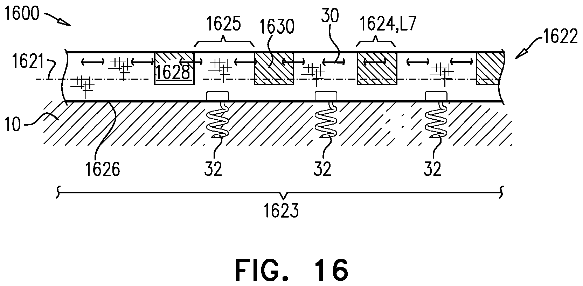

Reference is now made to Fig. 16, which is a schematic illustration of a system 1600 comprising a structure 1622 (e.g., an annuloplasty structure, an annuloplasty ring structure, etc.) having a flexible body portion 1623 (e.g., an annuloplasty body portion, an annuloplasty ring body portion, etc.) comprising sleeve 1626 and a contracting member 30, in accordance with some applications. Sleeve 1626 of body portion 1623 comprises a first flexible material and is shaped and configured so as to define a plurality of windows 1628 which each covered by a respective cover 1630 comprising a second, flexible material that is more stretchable and/or compressible than the first flexible material of sleeve 1626. Sleeve 1626 thereby defined a plurality of anchor-designated sections or attachment sections 1625 alternating along a longitudinal axis 1621 of structure 1622 with a plurality of compressible sections 1624 defined by windows 1628. As shown, a respective tissue anchor 32 can anchor structure 1622 to annulus tissue 10 at each anchor-designated section 1625.

In some applications, each window 1628, and each compressible section 1624 has a longest dimension L7 of 12-16 mm, e.g., 14 mm.

Reference is now made to Figs. 10 and 16. For some applications, each cover 1630 of Fig. 16 can comprise the same material of first longitudinal section 730 and can comprise flexible material comprising a plurality of interwoven fibers. First and second portions 750 and 752 of the interwoven fibers imparts increased longitudinal compressibility and/or stretchability to first longitudinal section 730, and thereby, to covers 1630 of Fig. 16. Each anchor-designated section 1625 can comprise the same material of second longitudinal section 732 which comprises flexible material comprising a plurality of interwoven fibers. Third and fourth portions 754 and 756 of the interwoven fibers imparts increased rigidity and/or stability to second longitudinal section 732, and thereby, to anchor-designated section 1625. For some applications, each section 1625 is labeled with a radiopaque marker.

Covers 1630, due to their respective interwoven fibers, have a greater degree of compressibility and/or stretchability than anchor-designated sections 1625. As such, windows 1628 and covers 1630 reduce the overall compressible force to body portion 1623 by contracting member 30 during contraction of structure 1622.

Reference is now made to Figs. 12 and 16. For some applications, each cover 1630 of Fig. 16 can comprise the same material of first section 932 of Fig. 12 which can define a compressible section having a first thickness T1 of 0.05-0.1 mm, e.g., 0.07 mm. Each anchor-designated section or attachment section 1625 can comprise the same material of second section 930 which can have a second thickness T2 of 0.15-0.2 mm, e.g., 0.18 mm. Second thickness T2 can be greater than first thickness T1 and second thickness T2 can be configured to impart rigidity and/or stability to the respective sections of sleeve 1626 though which a respective anchor is deployed. For some applications, each section 1625 is labeled with a radiopaque marker.

Covers 1630, due to their respective thickness, have a greater degree of compressibility than anchor-designated sections 1625. As such, windows 1628 and covers 1630 reduce the overall compressible force to body portion 1623 by contracting member 30 during contraction of structure 1622.

Reference is again made to Fig. 16. Sleeve 1626 can comprise a braided fabric mesh, e.g., comprising polyethylene terephthalate, Dacron (TM), other materials, etc. Body portion 1623 (e.g., sleeve 1626) can be configured to be placed only partially around a cardiac valve annulus (i.e., to assume a C-shape), and, once anchored in place, to be contracted so as to circumferentially tighten the valve annulus. Optionally, the body portion can be configured to be placed entirely around the valve annulus. In order to tighten the annulus, the system comprises an actuatable adjustment mechanism or contracting mechanism. The adjustment mechanism or contracting mechanism can be part of and/or coupled to one or more of the annuloplasty structure 1622, a flexible elongate contracting member 30 that extends along sleeve 1626, and/or another component. Elongate contracting member 30 can comprise a wire, a ribbon, a rope, or a band, which can comprise a flexible and/or superelastic material, e.g., nitinol, polyester, stainless steel, or cobalt chrome. For some applications, the contracting member 30 comprises a radiopaque material. For some applications, contracting member 30 comprises a braided polyester suture (e.g., Ticron). For some applications, contracting member 30 is coated with polytetrafluoroethylene (PTFE). For some applications, contracting member 30 comprises a plurality of wires that are intertwined to form a rope structure. The actuatable adjustment mechanism or contraction mechanism can be configured in a variety of ways, such as in any of the ways described elsewhere herein with respect to other actuatable adjustment mechanisms and/or in other documents.

Once the physician has positioned structure 1622 along the annulus and has anchored or attached structure 1622 to the annulus, contracting member 30 is used to contract annuloplasty structure 1622. For some applications, contracting member 30 is coupled to and/or is part of an actuatable contracting mechanism or adjustment mechanism, for example, as described elsewhere herein and/or as described in US Patent Application Publication 2014/0309661 to Sheps et al., and/or in US Patent Application Publication 2015/0272734 to Sheps et al.. Additionally, implantation of structure 922 can be done using any methods described in the `661 and/or `734 applications.

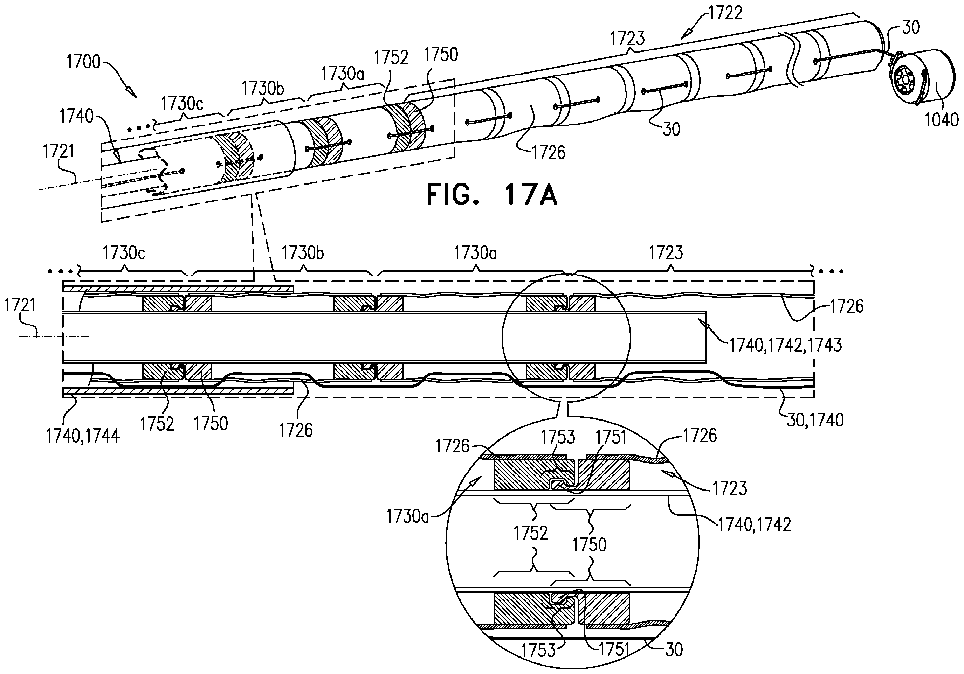

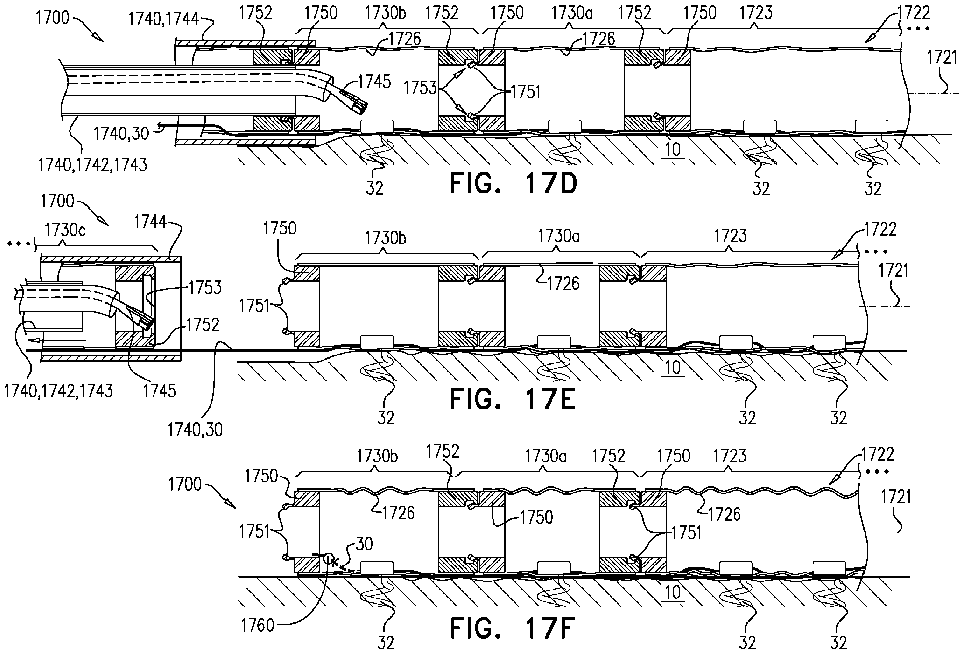

Reference is now made to Figs. 17A-F, which are schematic illustrations of a system 1700 comprising a structure 1722 (e.g., an annuloplasty structure, an annuloplasty ring structure, etc.) having a primary body portion 1723 comprising a flexible sleeve 1726 and a contracting member 30, in accordance with some applications. Sleeve 1726 of body portion 1723 comprises a flexible material shaped and configured so as to define a lumen therethrough. Structure 1722 comprises one or more linking segments 1730, e.g., 1730a, 1730b, and 1730c, as shown. Structure 1722 is shown as comprising three segments 1730 by way of illustration and not limitation. It is to be noted that structure 1722 can comprise any number of segments 1730. Segments 1730 are removably coupled to primary body portion 1723.

Structure 1722 is provided comprising any number of linking segments 1730 coupled to primary body portion 1723 such that structure 1722 can be delivered to the annulus of the patient in an elongate state and, subsequently, any number of linking segments 1730 can be decoupled from primary body portion 1723. Once a sufficient amount of structure 1722 (i.e., primary body portion 1723 and any number of linking segments 1730) is anchored to annulus tissue 10, the physician can choose to decouple and remove from the patient's body the remaining linking segments 1730. In such a manner, system 1700 minimizes the likelihood of any excess sleeve 1726 interfering with the cardiac valve. Additionally, with system 1700, the physician does not need to measure the size of the annulus prior to positioning annuloplasty ring structure 1722. In such a manner, structure 1722 accommodates and fits all native annulus sizes.

Primary body portion 1723 as well as linking segments 1730 comprise flexible sleeve 1726.

Sleeve 1726 can comprise a braided fabric mesh, e.g., comprising polyethylene terephthalate, e.g., Dacron (TM). Body portion 1723 (e.g., sleeve 1726) can be configured to be placed only partially around a cardiac valve annulus (i.e., to assume a C-shape), and, once anchored in place, to be contracted so as to circumferentially tighten the valve annulus. Optionally, the body portion can be configured to be placed entirely around the valve annulus. In order to tighten the annulus, the system comprises an actuatable adjustment mechanism or contracting mechanism. The adjustment mechanism or contracting mechanism can be part of and/or coupled to one or more of the annuloplasty structure 1722, a flexible elongate contracting member 30 that extends along sleeve 1726, and/or another component. Elongate contracting member 30 can comprise a wire, a ribbon, a rope, or a band, which can comprise a flexible and/or superelastic material, e.g., nitinol, polyester, stainless steel, or cobalt chrome. For some applications, the contracting member 30 comprises a radiopaque material. For some applications, contracting member 30 comprises a braided polyester suture (e.g., Ticron). For some applications, contracting member 30 is coated with polytetrafluoroethylene (PTFE). For some applications, contracting member 30 comprises a plurality of wires that are intertwined to form a rope structure. The actuatable adjustment mechanism or contraction mechanism can be configured in a variety of ways, such as in any of the ways described elsewhere herein with respect to other actuatable adjustment mechanisms and/or in other documents.

Contracting member 30 extends along a longitudinal length of primary body portion 1723 as well as along each of linking segments 1730. Contracting member 30 can be used to contract primary body portion 1723 as well as linking segments 1730 along a longitudinal axis 1721 of structure 1722.

A first end, e.g., a proximal end of primary body portion 1723 can comprise a first coupling 1750. A second coupling 1752 can be coupled to a first end, e.g., a distal end, of a first linking segment 1730a that is disposed adjacent to first coupling 1750 of the first end of primary body portion 1723. First and second couplings 1750 and 1752 are shaped and configured so as to mate with each other.

A third coupling (e.g., another coupling 1750) can be coupled to a second end, e.g., a proximal end, of first linking segment 1730a. A fourth coupling (e.g., another coupling 1752) can be coupled to a first end (e.g., a distal end) of a second linking segment 1730b that is disposed adjacent to first linking segment 1730a.

First coupling 1750 is shaped and configured so as to define a first lumen and comprises one or more radially-moveable projections 1751. Second coupling 1752 is shaped and configured so as to define a second lumen and a negative space 1753 shaped and configured so as to receive one or more radially-moveable projections 1751.

For some applications, radially-moveable projection 1751 of first coupling 1750 comprises a single, circular projection and negative space 1753 of second coupling 1752 is shaped and configured so as to define a single, circular groove.

For some applications, radially-moveable projections 1751 are biased to collapse radially inwardly in an absence of force applied thereto. For some applications, radially-moveable projections 1751 are not biased to collapse radially inwardly, and are moveable in response to a force applied thereto. For example, the walls defining negative space 1753 of second coupling 1752 are shaped and configured so as to enable slidable decoupling of the one or more projections 1751 in one direction, in response to a force applied to linking segment 1730, e.g., a pulling force applied to linking segment 1730.

System 1700 comprises a decoupling-prevention element 1740, as shown in Fig. 17A. Decoupling-prevention element extends longitudinally along at least first and second couplings 1750 and 1752 in order to prevent decoupling of first and second couplings 1750 and 1752. During delivery of structure 1722, decoupling-prevention element 1740 extends along all of linking segments 1730 as well as along a portion of a primary body portion 1723 and prevents decoupling of linking segments 1730 from each other as well as from primary body portion 1723.

For some applications, decoupling-prevention element 1740 comprises an elongate tube 1742. The external surface of tube 1742 is sized so as to rest against or push against one or more projections 1751 so as to maintain projections 1751 within negative space 1753 and prevent decoupling of first and second couplings 1750 and 1752.

For applications in which one or more projections 1751 are biased to move radially inward, the external surface of tube 1742 of decoupling-prevention element 1740 pushes against one or more projections 1751 of first coupling 1750 and maintains projections 1751 within negative space 1753 of second coupling 1752 in order to prevent decoupling of first and second couplings 1750 and 1752 while a portion of elongate tube 1742 passes through the first and second lumens of first and second couplings 1750 and 1752, respectively.

For some applications, tube 1742 of decoupling-prevention element 1740 comprises a tube of an anchor-delivery system 1743. For some applications, anchor-delivery system 1743 comprises the deployment manipulator, anchor driver, and deployment element, as described in US Patent Application Publication 2014/0309661 to Sheps et al., and/or in US Patent Application Publication 2015/0272734 to Sheps et al..

For some applications, decoupling-prevention element 1740 comprises an overtube 1744, e.g., a steerable catheter, which is used to deliver annuloplasty structure 1722. Overtube 1744 has an inner diameter sized so as to maintain coupling of couplings 1750 and 1752 when a portion of overtube 1744 surrounds first and second couplings 1750 and 1752.

For some applications, decoupling-prevention element 1740 comprises contracting member 30. Since contracting member 30 extends along, e.g., threaded along, primary body portion 1723 and linking segments 1730, contracting member 30 maintains coupling of first and second couplings 1750 and 1752.

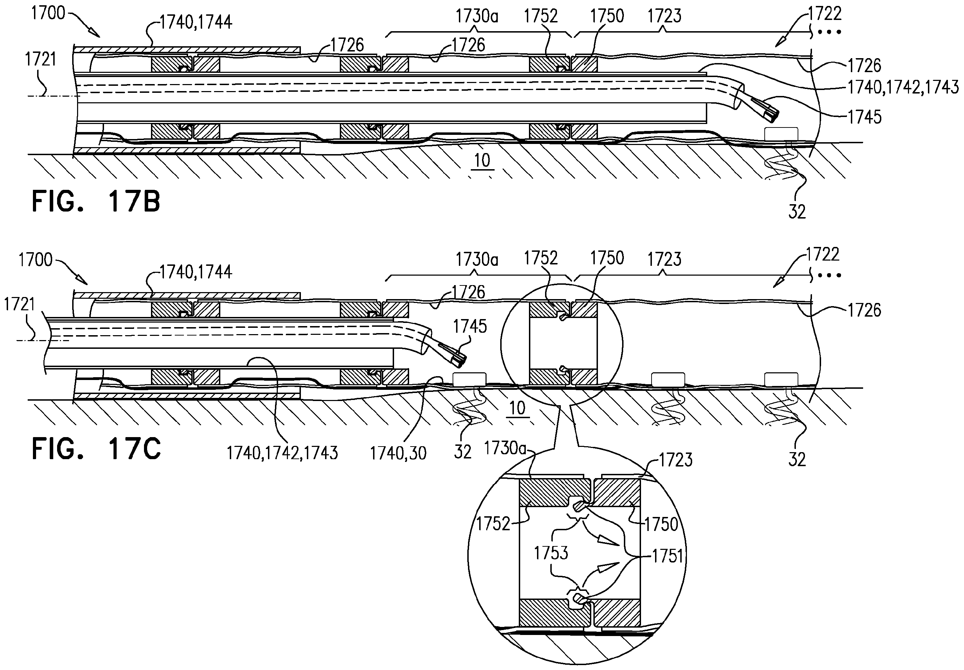

Fig. 17B shows anchor-delivery system 1743 delivering a tissue anchor 32 through a portion of sleeve 1726 of primary body portion 1723 and into cardiac tissue 10. System 1743 comprises an anchor driver 1745 which is reversibly coupled to anchor 32 and is decoupled therefrom following deployment of anchor 32.

Once primary body portion 1723 is anchored to cardiac tissue 10, anchor-delivery system 1743 is moved in order to position first linking segment 1730a appropriately at cardiac tissue 10 to be anchored thereto. As shown in Fig. 17C, a portion of tube 1742 of system 1743 is moved away from first and second couplings 1750 and 1752.

For applications in which one or more projections 1751 of first coupling 1750 is biased to move radially inward, in the absence of decoupling-prevention element 1740, projections 1751 move radially inward, as shown in Fig. 17C.

It is to be noted that for some applications, one or more projections 1751 of first coupling 1750 are not biased to move radially inward, and are only moveable in response to a force applied thereto, e.g., a pulling force applied to segment 1730a from a proximal direction. In such applications, one or more projections 1751 do not move radially inwardly in the absence of the pushing force applied thereto by the external surface of tube 1742.

In either application, contracting member 30 maintains coupling of linking segment 1730a and primary body portion 1723 even though tube 1742 is not disposed within the respective elements of couplings 1750 and 1752 and, as a result, one or more projections 1751 are moveable from within negative space 1753. In such a manner, contracting member 30 functions as decoupling-prevention element 1740. Additionally, since segment 1730a is anchored to tissue 10, coupling of segment 1730 and primary body portion 1723 is maintained.

In Fig. 17D, second linking segment 1730b is anchored to cardiac tissue 10 by anchor delivery system 1743, in a manner as described hereinabove with reference to Fig. 17C. Accordingly, since tube 1742 of decoupling-prevention element 1740 extends beyond the lumens of couplings 1750 and 1752 coupling first and second linking segments 1730a and 1730b, one or more projections 1751 of coupling 1750 of first linking segment 1730a is moveable from within negative space 1753 of coupling 1752 of second linking segment 1730b.

As noted hereinabove with reference to Fig. 17C, for applications in which one or more projections 1751 of first coupling 1750 is biased to move radially inward, in the absence of decoupling-prevention element 1740, projections 1751 move radially inward.

Additionally, as noted in Fig. 17C, it is to be noted that for some applications, one or more projections 1751 of first coupling 1750 are not biased to move radially inward, and are only moveable in response to a force applied thereto, e.g., a pulling force applied to segment 1730a from a proximal direction. In such applications, one or more projections 1751 do not move radially inwardly in the absence of the pushing force applied thereto by the external surface of tube 1742.

Fig. 17E shows decoupling of third linking segment 1730c from second linking segment 1730b.

As noted hereinabove with reference to Fig. 17C, for applications in which one or more projections 1751 of first coupling 1750 is biased to move radially inward, in the absence of decoupling-prevention element 1740, projections 1751 move radially inward.

Additionally, as noted in Fig. 17C, it is to be noted that for some applications, one or more projections 1751 of first coupling 1750 are not biased to move radially inward, and are only moveable in response to a force applied thereto, e.g., a pulling force applied to segment 1730c from a proximal direction. In such applications, one or more projections 1751 do not move radially inwardly in the absence of the pushing force applied thereto by the external surface of tube 1742.

In either application, third linking segments 1730c can be decoupled from second linking segment following radial movement of one or more projections 1751 of coupling 1750 of second linking segment 1730b from within negative space 1753 of coupling 1752 of third linking segment 1730c.

As shown, third linking segment 1730c, as well as any other additional linking segments 1730 disposed proximally to third linking segment 1730c, are moved proximally away from primary body portion 1723 and first and second linking segments 1730a and 1730b coupled to cardiac tissue. As shown, third linking segment 1730c, as well as any other additional linking segments 1730 disposed proximally to third linking segment 1730c are pulled along contracting member 30.

Fig. 17E shows structure 1722 following contracting thereof. After third linking segment 1730c, as well as any other additional linking segments 1730 disposed proximally to third linking segment 1730c are removed from the body of the subject, excess portions of contracting member 30 are cut and removed from the body of the patient. A bead or lock 1760 is coupled to contracting member 30.

Once the physician has positioned structure 1722 along the annulus and has removed any number of linking segments 1730, contracting member 30 can be used to contract annuloplasty structure 1722, for example, by actuating contracting mechanism 1040, which comprises and/or is coupled to contracting member 30 as described in US Patent Application Publication 2014/0309661 to Sheps et al., and/or in US Patent Application Publication 2015/0272734 to Sheps et al.. Additionally, implantation of structure 1722 can be done using any methods described in the `661 and/or `734 applications.

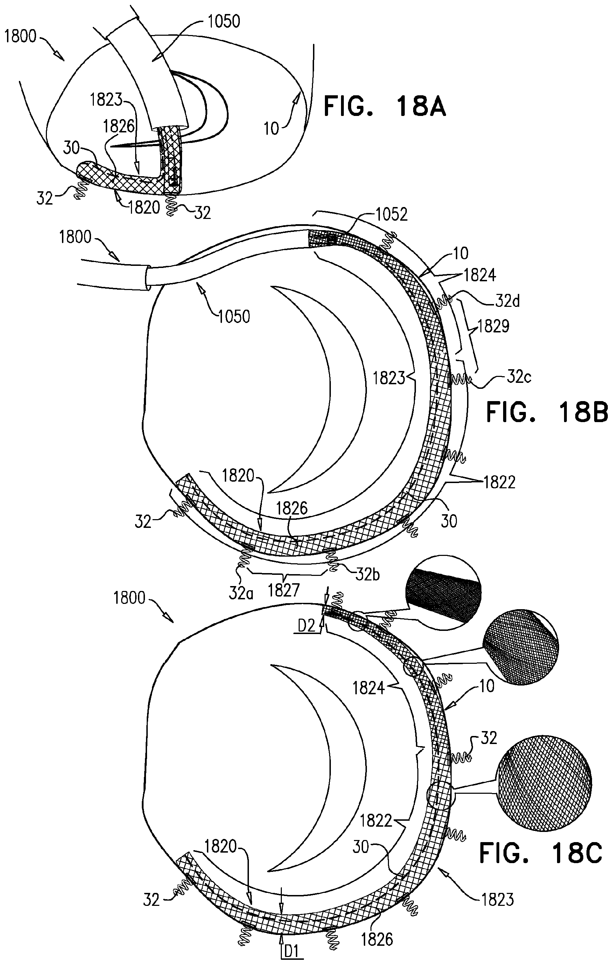

Reference is now made to Figs. 18A-C, which are schematic illustrations of an exemplary system 1800 comprising a variably-stretchable structure 1820 (e.g., an annuloplasty structure, an annuloplasty ring structure, etc.) having a flexible body portion 1823 (e.g., an annuloplasty body portion, an annuloplasty ring body portion, etc.) comprising a sleeve 1826 and a contracting member 30, in accordance with some applications. Annuloplasty structure 1820 can comprise any annuloplasty structure or annuloplasty ring structure described herein with reference to Figs. 1-17F. Additionally, for some applications, structure 1820 can comprise an actuatable contracting mechanism comprising and/or coupled to contracting member 30 as described in US Patent Application Publication 2014/0309661 to Sheps et al., and/or in US Patent Application Publication 2015/0272734 to Sheps et al.. Additionally, implantation of structure 1820 can be done using any methods described in the `661 and/or `734 applications.

Sleeve 1826 can comprise a braided fabric mesh, e.g., comprising polyethylene terephthalate, Dacron (TM), other materials, etc. Body portion 1823 (e.g., sleeve 1826) can be configured to be placed only partially around a cardiac valve annulus (i.e., to assume a C-shape), and, once anchored in place, to be contracted so as to circumferentially tighten the valve annulus. Optionally, the body portion can be configured to be placed entirely around the valve annulus (e.g., a full or closed ring). In order to tighten the annulus, the system comprises an actuatable adjustment mechanism or contracting mechanism. The adjustment mechanism or contracting mechanism can be part of and/or coupled to one or more of the annuloplasty structure 1820, a flexible elongate contracting member 30 that extends along sleeve 1826, and/or another component. Elongate contracting member 30 can comprise a wire, a ribbon, a rope, or a band, which can comprise a flexible and/or superelastic material, e.g., nitinol, polyester, stainless steel, or cobalt chromium. For some applications, the contracting member 30 comprises a radiopaque material. For some applications, contracting member 30 comprises a braided polyester suture (e.g., Ticron). For some applications, contracting member 30 is coated, such as with polytetrafluoroethylene (PTFE) or another polymer. For some applications, contracting member 30 comprises a plurality of wires that are intertwined to form a rope structure. The actuatable adjustment mechanism or contraction mechanism can be configured in a variety of ways, such as in any of the ways described elsewhere herein with respect to other actuatable adjustment mechanisms and/or in other documents.

For some applications, sleeve 1826 is labeled with one or more radiopaque markers. Sleeve 1826 can comprise a flexible sleeve 1826 shaped and configured so as to define a lumen therethrough.

In Fig. 18A, an anchor-delivery system 1050 or other attachment system can be used to anchor or attach a first section of annuloplasty body portion 1823 of annuloplasty structure 1820, for example, by deploying one or more tissue anchors 32 through sleeve 1826 and into annulus tissue 10, from within a lumen of sleeve 1826. Techniques for deploying anchors 32 through sleeve 1826 can be practiced in combination with techniques described in WO 2013/069019 to Sheps. Other fastening or attachment means (e.g., staples, fasteners, clips, screws, adhesives, etc.) can also or alternatively be used.

In Fig. 18B, anchor-delivery system 1050 is used to deliver and anchor or attach additional sections of body portion 1823 of annuloplasty structure 1820. As shown, body portion 1823 comprises (1) a first segment 1822 having a first degree of stretchability, and (2) a second segment 1824 having a second degree of stretchability that is greater than the first degree of stretchability. For some applications, system 1050 can reversibly engage and reversibly grip to a portion of sleeve 1826 during the stretching of second segment 1824, e.g., in order to facilitate pulling of sleeve 1826.

A respective length of each of first and second segments 1822 and 1824 can be controlled by the operating physician.

For some applications, the entire body portion 1823 has a gradually increasing flexibility from a distal end of body portion 1823 toward a proximal end of body portion 1823. That is, second segment 1824 can be longer and/or assume a greater percentage of body portion 1823 than as shown in Figs. 18B-C by way of illustration and not limitation.

In the context of the present application, including the claims "proximal" refers to a part of the structure that is closer to the orifice through which the structure was introduced and "distal" refers to a part of the structure that is farther from the orifice through which the structure was introduced.

Additionally, for some applications, the increased stretchability of segment 1824 imparts increased compressibility to segment 1824. For example, body portion 1823 can be delivered to the heart in an elongate state, anchoring in part to the annulus, and then subsequently to the anchoring, shortening of the elongate structure (e.g., by compressing) in real time during the implantation procedure while maintaining the body portion of the structure or annuloplasty ring structure intact (e.g., by not cutting or severing any portion of the body portion), and subsequently to the shortening, contracting of the structure or annuloplasty ring structure by contracting member 30 in order to contract and remodel the annulus of the patient. For example, for some applications, after having fixed first segment 1822 to a first length of tissue of the annulus, it may become apparent to the physician that the length of path of the remaining tissue of the annulus is shorter than initially anticipated. With structure 1820 having segment 1824 with increased compressibility, the physician can the compress the remaining sections of second segment 1824 in order to accommodate the length of the path of the remaining tissue of the annulus.

Once the physician has fixed (e.g., typically by anchoring with anchor 32 and/or fastening with some other attachment means) first segment 1822 to the annulus, the physician subsequently stretches second segment 1824 in order to accommodate the remaining portions of the annulus to which structure 1820 has not yet been anchored. For example, for some applications, after having fixed first segment 1822 to a first length of tissue of the annulus, it may become apparent to the physician that the length of path of the remaining tissue of the annulus is longer than initially anticipated. With structure 1820 having segment 1824 with increased stretchability, the physician can the stretch second segment 1824 in order to accommodate the length of the path of the remaining tissue of the annulus. Subsequently to the stretching of second segment 1824, second segment 1824 is fixed to the annulus (e.g., one or more anchors 32 are deployed through sleeve 1826 of second segment 1824 in order to fix second segment 1824 to the annulus). In such a manner, a shorter annuloplasty structure can be delivered to the annulus and only stretched to a longer length once initially anchored to the annulus. As such, structure 1820 comprises a partially-stretchable, or partially-elastic structure 1820. For some applications, as body segment 1824 of body portion 1823 is stretched, a diameter of segment 1824 incrementally and gradually decreases.

As shown in Figs. 18B-C, first segment 1822 is designated for implantation between the vicinity of the anterolateral commissure and roughly P2 (by way of illustration and not limitation), while second segment 1824 is designated for implantation between roughly P2 (by way of illustration and not limitation) and the posterolateral commissure. Fig. 18C shows the various diameters of sleeve 1826. It is to be noted that although only second segment 1824 is shown as being more stretchable than first segment 1822, applications include an annuloplasty structure having a body portion that is has alternating segments of variable stretchability. Segment 1824 can be designated for implantation anywhere along the annulus.

Since first segment 1822 has a degree of stretchability that is lower than second segment 1824, first segment 1822 typically but not necessarily has a D1 greater than a diameter D2 of second segment 1824 following stretching, and typically before contraction of structure 1820.

For some applications, the diameter of segment 1824 does not decrease following stretching. In such applications, the diameter of segment 1824 remains the same or similar to the diameter of segment 1822. For some applications, only the inner diameter may be reduced in response to the stretching of segment 1824. For some applications, only the outer diameter may be reduced in response to the stretching of segment 1824. For either of these applications, appropriate materials can be used which help to control the diameter of a given section of structure 1820 responsively to the application of a pulling, stretching, or compressing force. These appropriate materials also help to control a thickness of the wall of sleeve 1826.

Typically, second segment 1824 is, overall, more stretchable than first segment 1822. For some applications, second segment 1824 is homogenous throughout. That means, that the entire segment 1824 is stretchable in equal measure along the entire length of segment 1824. For some applications, second segment 1824 can have sections that are more stretchable and sections which are less stretchable. For such applications, second segment 1824 can have increasing stretchability along its length, or, second segment 1824 can have sections of variable stretchability which alternate along the length of second segment 1824.

As shown by way of illustration and not limitation in Figs. 18B-C, second segment 1824 assumes roughly one-third of body portion 1824. It is to be noted that segments 1822 and 1824 can assume any proportion. For example, section 1824 can assume roughly 50% or 75% of body portion 1823. For some applications, body portion 1823 can be increasingly stretchable along the entire length of body portion 1823.

Although Figs. 18A-C shows sleeve 1826 comprising a braided fabric, sleeve 1826 can comprise other materials such as a flexible, tubular plastic, for example, that is variably-stretchable, or variably-elastic. For some applications, the sleeve can comprise a spiral design, for example, the sleeve can comprise flexible, tubular plastic that has a spiral design. For some applications, the spiral can have a variable pitch and/or diameter to help vary the flexibility of the sleeve in different regions.

Reference is now made to Figs. 18A-C. For first segment 1822, (1) anchors 32 (and/or another attachment means) can be deployed at an equal or similar distance from each other (e.g., a distance of 4-10 mm between each anchor/attachment means), and (2) an equal or similar length of sleeve 1826 can be disposed between each anchor/attachment means. For second segment 1824, (1) anchors 32 (or other attachment means) can be deployed at an equal or similar distance from each other, and (2) because sleeve 1826 is stretched, a smaller length of sleeve 1826 can be used to span the distance between each anchor 32 in second segment 1824 than the length of sleeve 1826 used to span the distance between anchors 32 in first segment 1822.

That is, for a method in which structure 1820 is implanted, first segment 1822 can be implanted by driving an anchor 32 through sleeve 1826 as shown in Fig. 18A (or using another attachment means). A first section of sleeve 1826 of first segment 1822 can be released from anchor delivery system 1050. The first section can then be positioned along tissue 10 and another anchor 32 can be driven through sleeve 1826 using an anchor driver 1052 in order to fix the first section of sleeve 1826 to tissue 10 (or this section can be fixed/attached to the tissue using another attachment means). Another section 1827 of sleeve 1826 of first segment 1822 can then be released from anchor delivery system 1050 and positioned along tissue 10 and another anchor 32a can be driven through sleeve 1826 (as is shown in Fig. 18B) (or the additional section 1827 can be fixed/attached to the tissue using another attachment means). Once the physician implants first segment 1822, the physician can expose a section 1829 of second segment 1824 by releasing section 1829 from delivery system 1050. Section 1829 of segment 1824 released from system 1050 has a length that is smaller than the length of section 1827 of segment 1822 released from system 1050. The physician can then stretch section 1829 to a stretched state having a length that is greater than the pre-stretched length of section 1829 released from delivery system 1050. The physician can position section 1829 in the stretched state along tissue 10 and can fix section 1829 of second segment 1824 to tissue 10, e.g., by an attachment means such as driving a tissue anchor 32d at a distance from the preceding anchor 32c that is equal or generally similar to the distance between anchors 32a and 32b used to anchor section 1827 of first segment 1822.

Since second segment 1824 has increased flexibility, (1) a smaller length of sleeve 1826 can be released from delivery system 1050 incrementally in order to span the distance between attachment means/anchors 32 used to fix second segment 1824, then (2) a length of sleeve 1824 can be released from delivery system 1050 in order to span the same distance between attachment means/anchors 32 of first segment 1822.

That is, if the physician stretches sleeve 1826 at section 1829 of second segment 1824 to the span same distance between each attachment means/anchor 32, a smaller length of sleeve 1826 is released from delivery system 1050 prior to stretching between each anchor 32 in second segment 1824 than the length of sleeve 1826 is released from delivery system 1050 in order to span the distance between anchors 32 in first segment 1822.

For some applications, the stretching of segment 1824 straightens sleeve 1826 prior to anchoring of sleeve 1826. This straightening of sleeve 1826 by stretching advantageously lowers the likelihood of folding of sleeve 1826 in the anchoring region, thereby minimizing interference of any folds of sleeve 1826 with anchors 32.

It is to be further noted, that even though sleeve 1826 is stretched at second segment 1824, the elastic force of second segment 1824 (i.e., the force exerted by sleeve 1826 in order to return to its original length following anchoring of segment 1824) can be selected and/or configured to be insufficiently strong to contract the annulus. Thereby, annuloplasty is only achieved when sleeve 1826 is intentionally contracted by the physician, e.g., using contracting member 30. That is, the elastic force of second segment 1824 is not strong enough to contract second segment 1824 enough that it will affect tissue 10 to which second segment 1824 is coupled.

Reference is again made to Figs. 18A-C. System 1800 minimizes the likelihood of any excess sleeve 1826 interfering with the cardiac valve. As shown in Fig. 18B, once first segment 1822 has been anchored, second segment 1824 is expanded by being stretched, and subsequently, anchors 32 are used to anchor second segment 1824 to the cardiac tissue. Additionally, with system 1800, the physician does not need to measure the size of the annulus prior to positioning annuloplasty structure 1820. In such a manner, structure 1820 accommodates and fits all native annulus sizes.

Once the physician has positioned structure 1820 along the annulus and has expanded segment 1824, contracting member 30 is used to contract annuloplasty structure 1820. For some applications, contracting member 30 is coupled to an actuatable contracting mechanism as described in US Patent Application Publication 2014/0309661 to Sheps et al., and/or in US Patent Application Publication 2015/0272734 to Sheps et al.. Additionally, implantation of structure 1820 can be done using any methods described in the `661 and/or `734 applications.

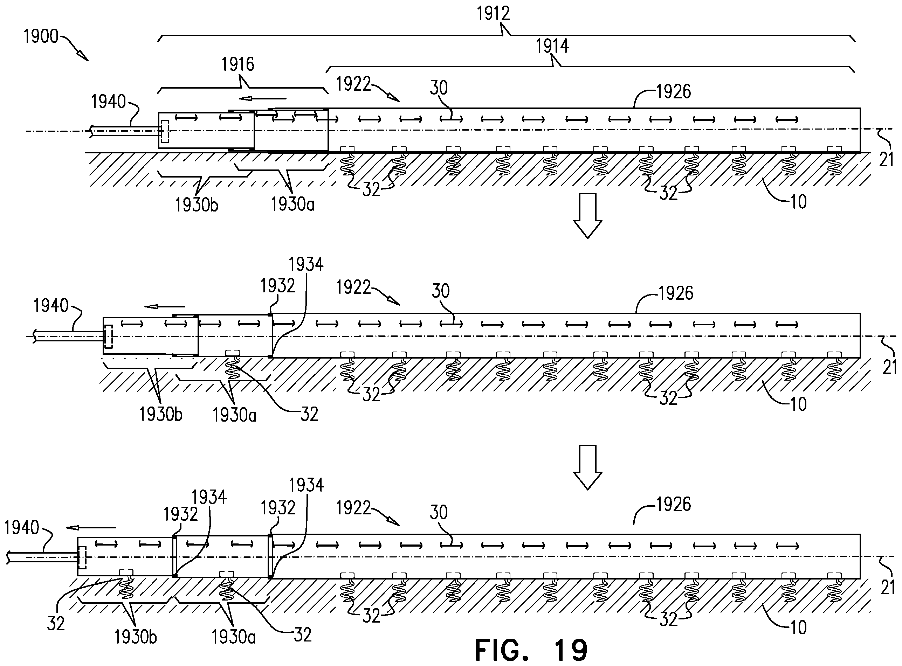

Fig. 19 is a schematic illustration of an exemplary system 1900 comprising a structure 1922 (e.g., an annuloplasty structure, an annuloplasty ring structure, etc.) having a body portion 1912 (e.g., an annuloplasty body portion, an annuloplasty ring body portion, etc.) comprising a sleeve 1926 and a contracting member 30, in accordance with some applications. For some applications, sleeve 1926 is tapered. Sleeve 1926 is shaped and configured so as to define a lumen therethrough. For some applications, body portion 1912 is flexible.

Structure 1922 comprises one or more (e.g., two as shown by way of illustration and not limitation) telescoping segments 1930a and 1930b. It is to be noted that structure 1922 can comprise any number of telescoping segments 1930 having any length as determined by a physician. For example, for some applications, all segments 1930 can have the same length. For other applications, segments 1930 can have a variable length. Structure 1922 can be delivered to the heart in a configuration in which telescoping segment 1930b is disposed at least in part within a lumen of telescoping segment 1930a, and telescoping segment 1930a is disposed at least in part within the lumen of sleeve 1926. Telescoping segments 1930a and 1930b can be moveable, or slidable, along a longitudinal axis 21 of body portion 1912.

A proximal end of sleeve 1926 comprises a coupling element 1934 which is configured to engage and become coupled to a coupling element 1932 at a distal end of telescoping segment 1930a. A proximal end of telescoping segment 1930a comprises a coupling element 1934 which is configured to engage and become coupled to a coupling element 1932 at a distal end of telescoping segment 1930b. For some applications, coupling elements 1932 and 1934 comprise hook-and-loop fasteners. For some applications, coupling elements 1932 and 1934 comprise magnets. Coupling elements 1932 and 1934 can comprise ring-shaped coupling elements which provide an unobstructed passage through the lumen of the entire structure 1922. Coupling elements 1932 and 1934 are coupled together typically following movement of the telescoping segment 1930 into its final position.

For some applications, telescoping segments 1930 comprise the same material as sleeve 1926. For some applications telescoping segments 1930 comprise material that is different from the material of sleeve 1826. For some applications, telescoping segments 1930 can have a wall thickness that is lower than the wall thickness of sleeve 1826.

Annuloplasty structure 1922 can comprise any annuloplasty structure or annuloplasty ring structure described herein with reference to Figs. 1-18C. Additionally, for some applications, structure 1922 can comprise an actuatable contracting mechanism comprising and/or coupled to contracting member 30 as described in US Patent Application Publication 2014/0309661 to Sheps et al., and/or in US Patent Application Publication 2015/0272734 to Sheps et al..

Sleeve 1926 and telescoping segments 1930 can comprise a braided fabric mesh, e.g., comprising polyethylene terephthalate, Dacron (TM), other materials, etc. Sleeve 1926 and telescoping segments 1930 can be configured to be placed only partially around a cardiac valve annulus (i.e., to assume a C-shape), and, once anchored in place, to be contracted so as to circumferentially tighten the valve annulus. Optionally, the ring structure can be configured to be placed entirely around the valve annulus (e.g., a full or closed ring). In order to tighten the annulus, the system comprises an actuatable adjustment mechanism or contracting mechanism. The adjustment mechanism or contracting mechanism can be part of and/or coupled to one or more of the annuloplasty structure 1922, a flexible elongate contracting member 30 that extends along sleeve 1926 and telescoping segments 1930, and/or another component. Elongate contracting member 30 can comprise a wire, a ribbon, a rope, or a band, which can comprise a flexible and/or superelastic material, e.g., nitinol, polyester, stainless steel, or cobalt chrome. For some applications, the contracting member 30 comprises a radiopaque material. For some applications, contracting member 30 comprises a braided polyester suture (e.g., Ticron). For some applications, contracting member 30 is coated, such as with polytetrafluoroethylene (PTFE) or another polymer. For some applications, contracting member 30 comprises a plurality of wires that are intertwined to form a rope structure. The actuatable adjustment mechanism or contraction mechanism can be configured in a variety of ways, such as in any of the ways described elsewhere herein with respect to other actuatable adjustment mechanisms and/or in other documents.

For some applications, sleeve 1926 and telescoping segments 1930 are labeled with one or more radiopaque markers. Typically, sleeve 1926 and telescoping segments 1930 comprises a flexible sleeve 1926 shaped and configured so as to define a lumen therethrough.

An anchor-delivery system 1940 or other attachment system can be used to anchor or attach a first segment 1914 of annuloplasty body portion 1912 of annuloplasty structure 1922, for example, by deploying one or more tissue anchors 32 through sleeve 1926 and into annulus tissue 10, from within a lumen of sleeve 1926. Techniques for deploying anchors 32 through sleeve 1926 can be practiced in combination with techniques described in WO 2013/069019 to Sheps.

As shown, body portion 1912 comprises (1) first segment 1912, and (2) a second segment 1916 comprising telescoping segments 1930. For some applications, anchor-delivery system 1940 can reversibly engage and reversibly grip to a portion of telescoping segments 1930 during the pulling and moving of telescoping segment 1930.

Any number of tissue anchors 32 or other fastening or attachment means can be used to anchor or attach structure 1922 to the annulus of the valve. In some embodiments, once a sufficient amount of first segment 1914 of body portion 1912 is anchored/attached to the annulus, the physician pulls and expands telescoping segment 1930a from within the lumen of sleeve 1926 using system 1940. Once telescoping segment 1930a has been pulled from within the lumen of sleeve 1926, it is anchored/attached to annulus tissue 10 using an attachment means/tissue anchor 32, as shown. The physician then pulls and expands telescoping segment 1930b from within the lumen of telescoping segment 1930a using system 1940. Once telescoping segment 1930b has been pulled from within the lumen of telescoping segment 1930a, it can be anchored/attached to annulus tissue 10 using an attachment means/tissue anchor 32, as shown. In such a manner, system 1900 minimizes the likelihood of any excess sleeve 1926 interfering with the cardiac valve. Additionally, with system 1900, the physician does not need to measure the size of the annulus prior to positioning annuloplasty ring structure 1922. In such a manner, structure 1922 accommodates and fits all native annulus sizes.

First segment 1914 can be designated for implantation between the vicinity of the anterolateral commissure and roughly P2, while second segment 1916 can be designated for implantation between roughly P2 and the posterolateral commissure. Telescoping segments 1930 can be located at any part of body portion 1912.

Once the physician has positioned structure 1922 along the annulus and has secured telescoping segments 1930, contracting member 30 is used to contract annuloplasty structure 1922. For some applications, contracting member 30 is coupled to an actuatable contracting mechanism as described in US Patent Application Publication 2014/0309661 to Sheps et al., and/or in US Patent Application Publication 2015/0272734 to Sheps et al.. Additionally, implantation of structure 1820 can be done using any methods described in the `661 and/or `734 applications.

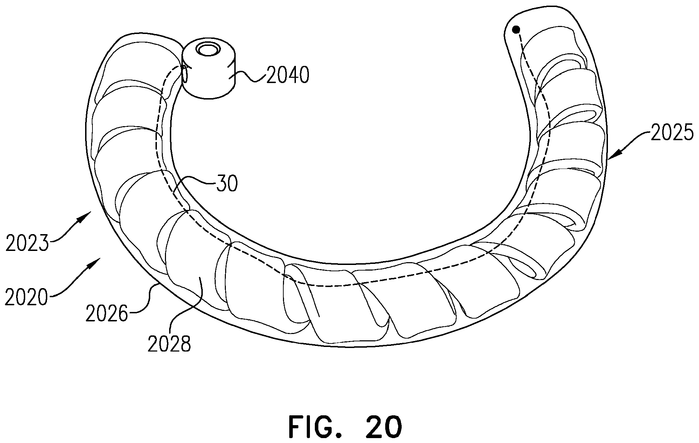

Reference is made to Figs. 20, 21A-B, 22A-C, and 23, which are schematic illustrations of an annuloplasty system 2000, and use thereof, in accordance with some applications. System 2000 comprises an elongate annuloplasty structure 2020 having a flexible body portion 2023 (e.g., an annuloplasty body portion, an annuloplasty ring body portion, etc.). Annuloplasty structure 2020 can comprise any annuloplasty structure or annuloplasty ring structure described herein. Typically, annuloplasty structure 2020 comprises contracting member 30 for contracting body portion 2023 after implantation of the annuloplasty structure. Contracting member 30 extends along body portion 2023. Additionally, for some applications, the system can comprise an actuatable adjustment mechanism 2040. The adjustment mechanism can be part of and/or coupled to one or more of the structure 2020, contracting member 30, and/or another component, e.g., as described elsewhere herein and/or as described in US Patent Application Publication 2014/0309661 to Sheps et al., and/or in US Patent Application Publication 2015/0272734 to Sheps et al.. Optionally, structure 2020 (and the other annuloplasty structures described herein) can be contracted by tensioning contracting member 30 by pulling on the contraction member, and using a locking element, such as a crimp, to lock in the tension. Implantation of structure 2020 can be performed using any methods described in the `661 and/or `734 applications.

Annuloplasty structure 2020 (e.g., body portion 2023) comprises a tubular wall 2025 that defines a lumen along body portion 2023. That is, the lumen is defined along a longitudinal axis of elongate annuloplasty structure 2020. Wall 2025 comprises a sleeve 2026 and a helical member 2028. Sleeve 2026 can comprise a braided fabric mesh, e.g., comprising polyethylene terephthalate, Dacron (TM), other materials, etc. Body portion 2023 (e.g., wall 2025, such as sleeve 2026) can be configured to be placed only partially around a cardiac valve annulus (i.e., to assume a C-shape), and, once anchored in place, to be contracted so as to circumferentially tighten the valve annulus. Optionally, the body portion can be configured to be placed entirely around the valve annulus (e.g., a full or closed ring). For some applications, body portion 2023 (e.g., sleeve 2026) is labeled with one or more radiopaque markers, e.g., as described hereinabove, *mutatis mutandis.*

Helical member 2028 defines a plurality of helical turns extending in a helical path along and around the lumen of body portion 2023. Helical member 2028 is coupled to sleeve 2026. For example, helical member 2028 (e.g., parts thereof, or the entirety thereof) can be stitched or glued to sleeve 2026. Structure 2020 is shown with helical member 2028 being disposed inside of sleeve 2026, but the helical member can optionally be disposed outside of the sleeve. For some applications, the helical member can be embedded within the sleeve.

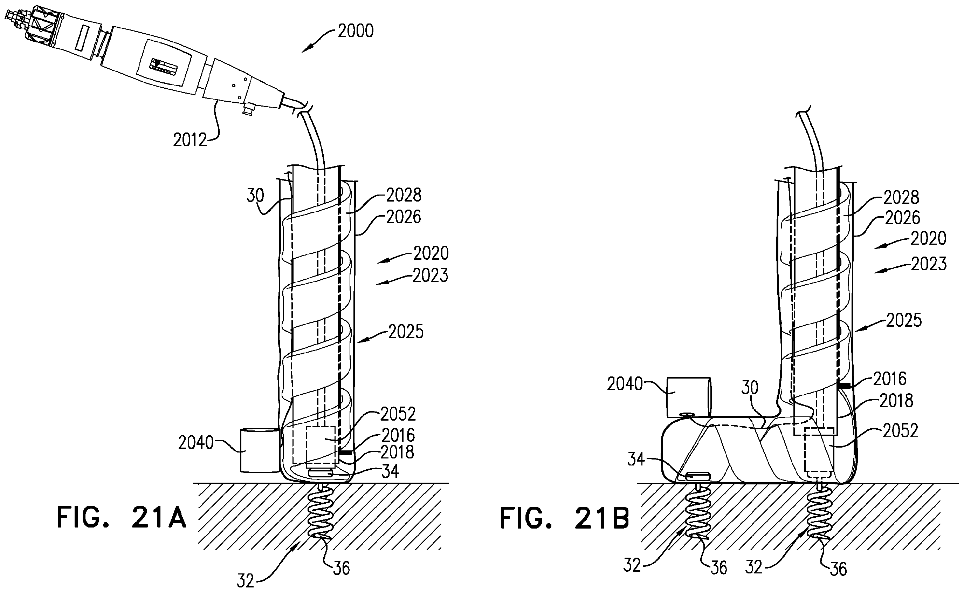

Many other annuloplasty structures described herein are described as being anchorable to tissue by anchors 32 being driven through the sleeve and into the tissue. Therefore, in some applications, such sleeves are configured to be sufficiently strong to support the resulting forces at the point of anchoring (e.g., at the point at which the anchor pierces the sleeve). As described hereinabove (e.g., with reference to Fig. 10), there is typically a trade-off between (i) strength, rigidity, and/or stability of a sleeve, and (ii) compressibility and/or stretchability of that sleeve. In order to provide increased longitudinal compressibility of body portion 2023 (e.g., so as to be adaptable to various annulus sizes), sleeve 2026 is made from a material (e.g., a fabric) that is particularly flexible, compressible, and/or stretchable (e.g., elastically stretchable). As a result, this material can be thinner, less dense, and/or weaker than that of other sleeves described herein. To accommodate this, body portion 2023 comprises helical member 2028, which is stronger, and typically stiffer than sleeve 2026. Annuloplasty structure 2020 is anchored to tissue by anchors 32 holding helical member 2028 to the tissue, typically by being driven through the helical member (Figs. 21A-B). Helical member 2028 thereby provides the strength required for anchoring annuloplasty structure 2020 and performing annuloplasty by contracting the annuloplasty structure, while sleeve 2026 continues to serve other functions that are served by other sleeves described herein, such as providing a closed lumen that reduces a likelihood of an anchor becoming an embolus.

For some applications, helical member 2028 comprises a polymer. For some applications, helical member 2028 is metallic. For some applications, helical member 2028 comprises a shape memory material. For some applications, helical member 2028 comprises a fabric.

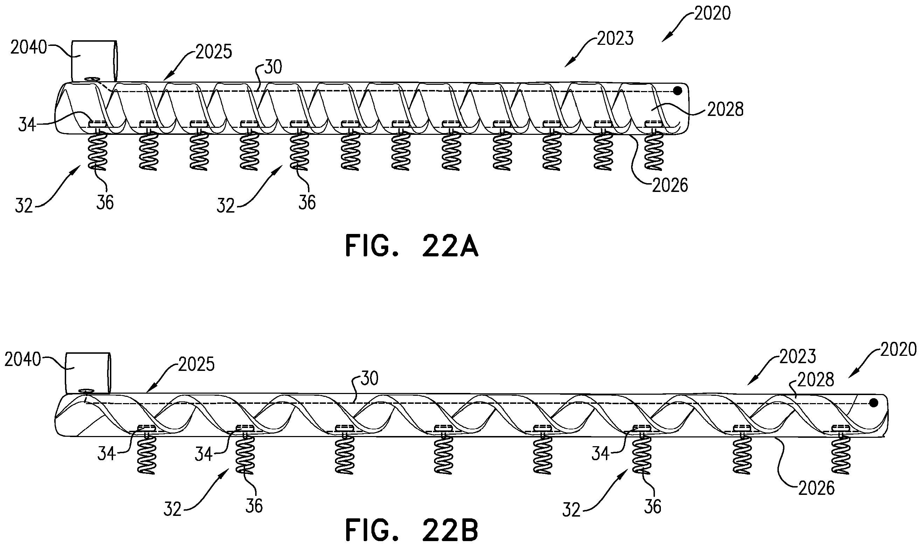

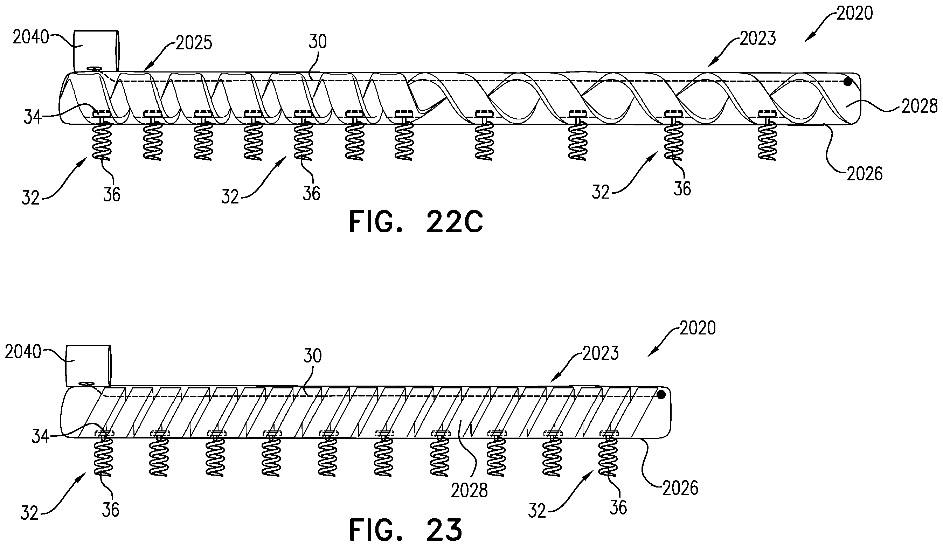

Helical member 2028 allows annuloplasty structure 2020 to be implanted relatively compressed for a relatively small annulus, with the helical member having a relatively small helix pitch (Fig. 22A), or relatively stretched for a relatively large annulus, with the helical member having a relatively small helix pitch (Fig. 22B). Furthermore, if during implantation, it is identified that the annulus is larger or smaller than originally understood, the operating physician can dynamically adapt the degree of compression/stretching for subsequently anchored parts of body portion 2023. Fig. 22C represents one example of such dynamic adaptation, in which, after seven anchors 38 had been anchored, it was identified that the annulus was larger than originally anticipated, and the operating physician dynamically increased the degree of stretching for the subsequent anchors.

For some other annuloplasty structures described herein, contracting member 30 extends along the body portion of the annuloplasty structure by weaving along the sleeve of the annuloplasty structure. In contrast, due to particular nature of sleeve 2026, for annuloplasty structure 2020 contracting member 30 extends through the lumen of body portion 2023, i.e., radially inward from helical member 2028.

Figs. 21A-B show steps in the implantation of annuloplasty structure 2020, in accordance with some applications. Attachment means, such as a plurality of anchors 38 can be used. In some implementations, each anchor 38 comprises a head 34 and a tissue-engaging element 36. Tissue-engaging element 36 is shown as a helical tissue-engaging element that is screwed into tissue, but can be any suitable type of tissue-engaging element, including a dart or a staple. Each anchor 32 is dimensioned to be advanceable along (i.e., longitudinally through) the lumen of body portion 2023, typically with tissue-engaging element 36 disposed distally to head 34. An anchor driver 2052 is reversibly couplable to anchor 32 (e.g., to head 34 thereof), and is configured to advance the anchor along the lumen (typically introducing the anchor into the lumen via an open proximal end of the lumen). In some implementations, anchor driver 2052 uses each anchor to secure a respective helical turn of helical member 2028 to the tissue by driving tissue-engaging element 36, from the lumen, through wall 2025, and into the tissue, such that the helical turn becomes sandwiched between head 34 and the tissue. As shown in Figs. 21A-B, tissue-engaging element 36 can be driven through helical member 2028 itself.

For some applications, and as shown in Fig. 23, tissue-engaging element 36 or at least some (or all) of the tissue-engaging elements can be driven through wall 2025 adjacent to the helical turn to be anchored (e.g., between helical turns), but sufficiently close to a helical turn that the helical turn becomes sandwiched between head 34 and the tissue. Fig. 23 shows adjacent helical turns being anchored by a single anchor 32 disposed between them. For some such applications, head 34 is wider than for applications in which tissue-engaging element 36 is driven through the helical member itself.

For some applications, every helical turn of helical member 2028 is secured to the tissue by a respective anchor 32 (e.g., as shown in Figs. 22A-C). For some applications, not every helical turn is secured by a respective anchor. For example, Fig. 21B shows a non-anchored helical turn disposed between two anchored helical turns.

Subsequent to its anchoring to the annulus, annuloplasty structure 2020 is contracted to reduce the size of the annulus, e.g., as described hereinabove, *mutatis mutandis.* Helical member 2028 and sleeve 2026 also facilitate this, with the helix pitch of the helical member becoming smaller during contraction.

For some applications, and as shown, system 2000 comprises a tool 2012 that facilitates delivery and/or anchoring of annuloplasty structure 2020. For some applications, anchor driver 2052 is a component of tool 2012. For some applications, tool 2012 comprises an anchor channel 2018 that extends into (and often through) the lumen of body portion 2023. For such applications, annuloplasty structure 2020 is typically delivered with channel 2018 already in place. Each anchor 32 is advanced into and through the lumen of body portion 2023 by being advanced through channel 2018. The anchor is typically anchored by advancing the anchor out of the distal opening of the channel and through the part of wall 2025 that is directly in front of the distal opening. After each part of body portion 2023 is anchored, a subsequent part of the body portion is advanced off of channel 2018 (e.g., by retracting channel 2018 with respect to the body portion), and that subsequent part is then anchored.

Control of this advancement can be facilitated by cooperation between channel 2018 and helical portion 2023. For example, when moving channel 2018 to the next tissue site to which annuloplasty structure 2020 is to be anchored, the resulting tension on body portion 2023 can tighten helical member 2028 around the channel, thereby gripping the channel, and increasing control over the advancement of the body portion off of the channel. For some applications, tool 2012 comprises a protrusion 2016 that extends radially outward from channel 2018, and engages helical member 2028, inhibiting the helical turn with which it engages from sliding off of the channel (Figs. 21A-B). Advancement of body portion 2023 off of channel 2018 can thereby be controlled by revolution of protrusion 2016 around a central longitudinal axis of the channel. For applications in which protrusion 2016 is rigidly fixed to channel 2018, this revolution is achieved by rotating channel 2018 about its longitudinal axis. Typically, one full revolution of protrusion 2016 (e.g., by one full rotation of channel 2018), optionally in combination with proximal retraction of channel 2018, allows one full turn of helical member 2028 (and the associated part of body portion 2023) to be advanced off of channel 2018.

For some applications, protrusion 2016 is movable with respect to channel 2018, thereby providing a further degree of adjustability and/or control over the advancement of body portion 2023 off of the channel. For example, and as shown in Figs. 21A-21B, protrusion 2016 can be longitudinally slidable with respect to channel 2018. Alternatively or additionally, protrusion 2016 can be revolvable circumferentially around channel 2018 independently of rotation of the channel itself.

Reference is now made to Figs. 1-23. Components of any system and/or apparatus shown or described herein can be combined with any other apparatuses or embodiments shown or described herein. For example, the fabric shown in Fig. 9 can be used in combination with structure 522 shown in Figs. 8A-B. Steps from the various methods shown or described herein can also be combined and arranged in a variety of ways.

Reference is again made to Figs. 1-23. Systems 20, 120, 220, 250, 320, 420, 620, 720, 820, 920, 1000, 1600, 1700, 1800, 1900, and 2000, and methods 1200 and 1300 for repairing a dilated annulus of the patient can be used to treat any cardiac valve of the patient, e.g., the aortic valve, the pulmonary valve, the mitral valve, and the tricuspid valve. Further, the methods, operations, steps, etc. described herein can be performed on a living animal or on a non-living cadaver, cadaver heart, simulator, anthropomorphic ghost, etc. Systems described herein for treatment of valves can be used to treat other annular muscles within the body of the patient. For example, the systems described herein can be used in order to treat a sphincter muscle within a stomach of the patient.

Reference is again made to Figs. 1-23. It is to be further noted that systems 20, 120, 220, 250, 320, 420, 620, 720, 820, 920, 1000, 1600, 1700, 1800, 1900, and 2000 can be anchored or attached to tissue (e.g., of the annulus) using any of the anchoring devices and/or attachment means described in US Patent Application Publication 2015/0272734 to Sheps et al., including the anchor driver and the deployment manipulator, or otherwise described herein.

Reference is again made to Figs. 1-23. The systems 20, 120, 220, 250, 320, 420, 620, 720, 820, 920, 1000, 1600, 1700, 1800, 1900, and 2000, and methods 1200 and 1300 (or subcomponents thereof) and methods described hereinabove can also be used on any suitable tissue of the patient (e.g., stomach tissue, urinary tract, and prostate tissue).

Additionally, some applications are described in one or more of the following:
- US Patent Application 12/435,291 to Maisano et al., entitled, "Adjustable repair chords and spool mechanism therefor," filed on May 4, 2009, which issued as US Patent 8,147,542;
- US Patent Application 12/437,103 to Zipory et al., entitled, "Annuloplasty ring with intra-ring anchoring," filed on May 7, 2009, which issued as US Patent 8,715,342;
- US Patent Application 12/548,991 to Maisano et al., entitled, "Implantation of repair chords in the heart," filed on August 27, 2009, which issued as US Patent 8,808,368;
- PCT Patent Application PCT/IL2009/001209 to Cabiri et al., entitled, "Adjustable annuloplasty devices and mechanisms therefor," filed on December 22, 2009, which published as PCT Publication WO 10/073246;
- PCT Patent Application PCT/IL2010/000357 to Maisano et al., entitled, "Implantation of repair chords in the heart," filed on May 4, 2010, which published as WO 10/128502;
- PCT Patent Application PCT/EL2010/000358 to Zipory et al., entitled, "Deployment techniques for annuloplasty ring and over-wire rotation tool," filed on May 4, 2010, which published as WO 10/128503;
- US Patent Application Publication 2014/0309661 to Sheps et al.; and/or
- US Patent Application Publication 2015/0272734 to Sheps et al.

Techniques described herein can be practiced in combination with techniques described in one or more of these applications.

It will be appreciated by persons skilled in the art that the present invention is not limited to what has been particularly shown and described hereinabove. Rather, the scope of the present invention includes both combinations and subcombinations of the various features and steps described hereinabove, as well as variations and modifications thereof that are not in the prior art, which would occur to persons skilled in the art upon reading the foregoing description as long as covered by the claims language.

## Claims

1. An apparatus, comprising:
an annuloplasty structure (22) comprising:
a body portion (23) comprising flexible material, the body portion (23) being shaped to define one or more controllably-expandable sections (40), each one of the one or more controllably-expandable sections (40) having a respective first length;
one or more control wires (50) coupled to the one or more controllably-expandable sections (40), the one or more control wires (50) being movable to facilitate expansion of each one of the one or more controllably-expandable sections (40) to assume a respective second length that is greater than the respective first length; and
a contracting member (30) coupled to the body portion (23), the contracting member (30) being configured to contract the body portion (23) independently of the one or more control wires (50).

2. The apparatus according to claim 1, wherein each one of the one or more controllably-expandable sections (40) comprises a part of the flexible material of the annuloplasty ring body portion (23), the part being folded, and wherein the one or more control wires (50) is threaded through folds of the part.

3. The apparatus according to claim 2, wherein the part is folded telescopically in a direction away from a longitudinal axis of the body portion (23) at the part.

4. The apparatus according to claim 2, wherein the part is folded in a manner in which at least one pouch (43) is formed between layers of folds of the part, and wherein the pouch (43) has a longitudinal length that is measured along a pouch (43) axis that is generally parallel with a longitudinal axis of the body portion (23) at the part.

5. The apparatus according to any one of claims 1 to 4, wherein the one or more control wires (50) is removably coupled to the one or more controllably-expandable sections (40), and wherein the flexible material is biased such that the one or more controllably-expandable sections (40) expand upon decoupling of the one or more control wires (50) from the one or more controllably-expandable sections (40).

6. The apparatus according to any one of claims 1 to 4, wherein the one or more control wires (50) is fixedly attached to the one or more controllably-expandable sections (40), and wherein the one or more control wires (50) facilitates expansion of the one or more controllably-expandable sections (40) responsively to pulling on the one or more control wires (50).

7. The apparatus according to any one of claims 1 to 6, wherein the one or more controllably-expandable sections (40) comprise a plurality of controllably-expandable sections (40).

8. The apparatus according to claim 7, wherein the one or more control wires (50) comprises exactly one control wire which controls all of the plurality of controllably-expandable sections (40).

9. The apparatus according to claim 7, wherein the one or more control wires (50) comprises a plurality of control wires, each one of the plurality of control wires (50) being movable to facilitate expansion of a respective one of the plurality of controllably-expandable sections (40).

10. A method performed on a simulation, comprising:
delivering an annuloplasty structure (22) to a simulated annulus of a simulated heart valve, the annuloplasty structure (22) including:
a body portion (23) comprising flexible material, the body portion (23) being shaped to define:
one or more controllably-expandable sections (40), each one of the one or more controllably-expandable sections (40) having a respective first length;
one or more control wires (50) coupled to the one or more controllably-expandable sections (40), the one or more control wires (50) being movable to expand each of the one or more controllably-expandable sections (40) to assume a respective second length that is greater than the respective first length; and
a contracting member (30) coupled to the body portion (23), the contracting member (30) being configured to contract the body portion (23) independently of the one or more control wires (50); and
controllably expanding the one or more controllably-expandable sections (40) by moving the one or more control wires (50).

## Patentansprüche

1. Gerät, umfassend:
eine Anuloplastik-Struktur (22), umfassend:
einen Körperabschnitt (23), umfassend flexibles Material, wobei der Körperabschnitt (23) so geformt ist, dass ein oder mehrere steuerbar expandierbare Abschnitte (40) definiert sind, wobei jeder von dem einen oder den mehreren steuerbar expandierbaren Abschnitten (40) eine jeweilige erste Länge hat;
einen oder mehrere Steuerdrähte (50), der/die an den einen oder die mehreren steuerbar expandierbaren Abschnitte (40) gekoppelt ist/sind, wobei der eine oder die mehreren Steuerdrähte (50) bewegbar ist/sind, um die Expansion von jedem von dem einen oder den mehreren steuerbar expandierbaren Abschnitten (40) zu erleichtern, um eine jeweilige zweite Länge anzunehmen, die größer als die jeweilige erste Länge ist; und
ein Kontraktionselement (30), das an den Körperabschnitt (23) gekoppelt ist, wobei das Kontraktionselement (30) ausgestaltet ist, um den Körperabschnitt (23) unabhängig von dem einen oder den mehreren Steuerdrähten (50) zu kontrahieren.

2. Gerät nach Anspruch 1, wobei jeder von dem einen oder den mehreren steuerbar expandierbaren Abschnitten (40) ein Teil des flexiblen Materials des Anuloplastik-Ringkörperabschnitts (23) umfasst/umfassen, wobei das Teil gefaltet ist, und wobei der eine oder die mehreren Steuerdrähte (50) durch Falten des Teils gefädelt ist/sind.

3. Gerät nach Anspruch 2, wobei das Teil teleskopartig in einer Richtung weg von der Längsachse des Körperabschnitts (23) an dem Teil gefaltet ist.

4. Gerät nach Anspruch 2, wobei das Teil in einer Weise gefaltet ist, in der mindestens eine Tasche (43) zwischen Schichten von Falten des Teils gebildet ist, und wobei die Tasche (43) eine Länge in Längsrichtung aufweist, die entlang einer Achse der Tasche (43) gemessen wird, die allgemein parallel zu einer Längsachse des Körperabschnitts (23) an dem Teil ist.

5. Gerät nach einem der Ansprüche 1 bis 4, wobei der eine oder die mehreren Steuerdrähte (50) entfernbar an den einen oder die mehreren steuerbar expandierbaren Abschnitte (40) gekoppelt ist/sind, und wobei das flexible Material so vorgespannt ist, dass der eine oder die mehreren steuerbar expandierbaren Abschnitte (40) beim Entkoppeln des einen oder der mehreren Steuerdrähte (50) von dem einen oder den mehreren steuerbar expandierbaren Abschnitten (40) expandiert/expandieren.

6. Gerät nach einem der Ansprüche 1 bis 4, wobei der eine oder die mehreren Steuerdrähte (50) fixiert an dem einen oder den mehreren steuerbar expandierbaren Abschnitten (40) befestigt ist/sind, und wobei der eine oder die mehreren Steuerdrähte (50) die Expansion des einen oder der mehreren steuerbar expandierbaren Abschnitte (40) in Reaktion auf das Ziehen an dem einen oder den mehreren Steuerdrähten (50) erleichtert/erleichtern.

7. Gerät nach einem der Ansprüche 1 bis 6, wobei der eine oder die mehreren steuerbar expandierbaren Abschnitte (40) eine Vielzahl von steuerbar expandierbaren Abschnitten (40) umfasst/umfassen.

8. Gerät nach Anspruch 7, wobei der eine oder die mehreren Steuerdrähte (50) genau einen Steuerdraht umfasst/umfassen, der alle von der Vielzahl der steuerbar expandierbaren Abschnitte (40) steuert.

9. Gerät nach Anspruch 7, wobei der eine oder die mehreren Steuerdrähte (50) eine Vielzahl von Steuerdrähten umfasst/umfassen, wobei jeder von der Vielzahl der Steuerdrähte (50) beweglich ist, um Expansion eines jeweiligen von der Vielzahl der steuerbar expandierbaren Abschnitte (40) zu erleichtern.

10. Verfahren, das an einer Simulation durchgeführt wird, umfassend:
Zuführen einer Anuloplastik-Struktur (22) zu einem simulierten Anulus einer simulierten Herzklappe, wobei die Anuloplastik-Struktur (22) einschließt:
einen Körperabschnitt (23), umfassend flexibles Material, wobei der Körperabschnitt (23) geformt ist zum Definieren von:
einem oder mehreren steuerbar expandierbaren Abschnitten (40), wobei jeder von dem einen oder den mehreren steuerbar expandierbaren Abschnitten (40) eine jeweilige erste Länge hat;
einen oder mehrere Steuerdrähte (50), der/die an den einen oder die mehreren steuerbar expandierbaren Abschnitte (40) gekoppelt ist/sind, wobei der eine oder die mehreren Steuerdrähte (50) bewegbar ist/sind, um jeden von dem einen oder den mehreren steuerbar expandierbaren Abschnitten (40) zu expandieren, um eine jeweilige zweite Länge anzunehmen, die größer als die jeweilige erste Länge ist; und
ein kontrahierendes Element (30), das an den Körperabschnitt (23) gekoppelt ist, wobei das Kontraktionselement (30) ausgestaltet ist, um den Körperabschnitt (23) unabhängig von dem einen oder den mehreren Steuerdrähten (50) zu kontrahieren; und
steuerbares Expandieren des einen oder der mehreren steuerbar expandierbaren Abschnitte (40) durch Bewegen des einen oder der mehreren Steuerdrähte (50).

## Revendications

1. Appareil comprenant :
une structure d'annuloplastie (22) comprenant :
une portion corps (23) comprenant un matériau flexible, la portion corps (23) étant façonnée pour définir une ou plusieurs sections déployables de manière régulable (40), chacune de ladite une ou desdites plusieurs sections déployables de manière régulable (40) présentant une première longueur respective ;
un ou plusieurs fils de commande (50) accouplés à ladite une ou auxdites plusieurs sections déployables de manière régulable (40), ledit un ou lesdits plusieurs fils de commande (50) étant mobiles pour faciliter le déploiement de chacune de ladite une ou desdites plusieurs sections déployables de manière régulable (40) afin d'adopter une seconde longueur respective qui est supérieure à la première longueur respective ; et
un élément de contraction (30) accouplé à la portion corps (23), l'élément de contraction (30) étant conçu pour contracter la portion corps (23) indépendamment dudit un ou desdits plusieurs fils de commande (50).

2. Appareil selon la revendication 1, chacune de ladite une ou desdites plusieurs sections déployables de manière régulable (40) comprenant une partie du matériau flexible de la portion corps annulaire d'annuloplastie (23), la partie étant pliée et ledit un ou lesdits plusieurs fils de commande (50) étant enfilés à travers des plis de la partie.

3. Appareil selon la revendication 2, la partie étant pliée de manière télescopique dans un sens s'éloignant d'un axe longitudinal de la portion corps (23) au niveau de la partie.

4. Appareil selon la revendication 2, la partie étant pliée d'une manière dans laquelle au moins une poche (43) est formée entre des couches de plis de la partie et la poche (43) présentant une longueur longitudinale qui est mesurée le long d'un axe de poche (43) qui est généralement parallèle à un axe longitudinal de la portion corps (23) au niveau de la partie.

5. Appareil selon l'une quelconque des revendications 1 à 4, ledit un ou lesdits plusieurs fils de commande (50) étant accouplés de manière amovible à ladite une ou auxdites plusieurs sections déployables de manière régulable (40) et le matériau flexible étant sollicité de telle sorte que ladite une ou lesdites plusieurs sections déployables de manière régulable (40) se déploient lors du désaccouplement dudit un ou desdits plusieurs fils de commande (50) de ladite une ou desdites plusieurs sections déployables de manière régulable (40).

6. Appareil selon l'une quelconque des revendications 1 à 4, ledit un ou lesdits plusieurs fils de commande (50) étant attachés de manière fixe à ladite une ou auxdites plusieurs sections déployables de manière régulable (40) et ledit un ou lesdits plusieurs fils de commande (50) facilitant le déploiement de ladite une ou desdites plusieurs sections déployables de manière régulable (40) en réponse à la traction sur ledit un ou lesdits plusieurs fils de commande (50).

7. Appareil selon l'une quelconque des revendications 1 à 6, ladite une ou lesdites plusieurs sections déployables de manière régulable (40) comprenant une pluralité de sections déployables de manière régulable (40).

8. Appareil selon la revendication 7, ledit un ou lesdits plusieurs fils de commande (50) comprenant exactement un fil de commande qui commande toutes les sections de la pluralité de sections déployables de manière régulable (40).

9. Appareil selon la revendication 7, ledit un ou lesdits plusieurs fils de commande (50) comprenant une pluralité de fils de commande, chacun de la pluralité de fils de commande (50) étant mobile pour faciliter le déploiement d'une section respective de la pluralité de sections déployables de manière régulable (40).

10. Procédé mis en oeuvre sur une simulation, comprenant :
la pose d'une structure d'annuloplastie (22) au niveau d'un anneau simulé d'une valvule cardiaque simulée, la structure d'annuloplastie (22) comprenant :
une portion corps (23) comprenant un matériau flexible, la portion corps (23) étant façonnée pour définir :
une ou plusieurs sections déployables de manière régulable (40), chacune de ladite une ou desdites plusieurs sections déployables de manière régulable (40) présentant une première longueur respective ;
un ou plusieurs fils de commande (50) accouplés à ladite une ou auxdites plusieurs sections déployables de manière régulable (40), ledit un ou lesdits plusieurs fils de commande (50) étant mobiles pour déployer chacune de ladite une ou desdites plusieurs sections déployables de manière régulable (40) afin d'adopter une seconde longueur respective qui est supérieure à la première longueur respective ; et
un élément de contraction (30) accouplé à la portion corps (23), l'élément de contraction (30) étant conçu pour contracter la portion corps (23) indépendamment dudit un ou desdits plusieurs fils de commande (50) ; et
le déploiement régulé de ladite une ou desdites plusieurs sections déployables de manière régulable (40) par déplacement dudit un ou desdits plusieurs fils de commande (50).
